(19) Europäisches Patentamt
European Patent Office
Office européen des brevets

(11) Publication number: 0 190 496
A2

(12) # EUROPEAN PATENT APPLICATION

(21) Application number: 85308936.5

(51) Int. Cl.[4]: C 07 D 211/22
C 07 D 405/12

(22) Date of filing: 09.12.85

(30) Priority: 13.12.84 GB 8431478
16.08.85 GB 8520619

(43) Date of publication of application:
13.08.86 Bulletin 86/33

(84) Designated Contracting States:
BE CH DE FR GB IT LI LU NL SE

(71) Applicant: BEECHAM GROUP PLC
Beecham House Great West Road
Brentford Middlesex TW8 9BD(GB)

(72) Inventor: Stemp, Jean Anne
107 Rundells
Harlow Essex, CM18 7HD(GB)

(72) Inventor: Miller, David
10 Thornfield Road
Bishop's Stortford Hertforshire CM23 2RB(GB)

(72) Inventor: Martin, Roger Thomas
472 Vardon Road
Stevenage Hertfordshire(GB)

(74) Representative: Jones, Pauline et al,
Beecham Pharmaceuticals Patents & Trade Marks Dept.
Great Burgh Yew Tree Bottom Road
Epsom Surrey KT18 5XQ(GB)

(54) **Piperidine derivatives having a gastro-intestinal activity.**

(57) Compounds of formula (I) and pharmaceutically acceptable salts thereof:

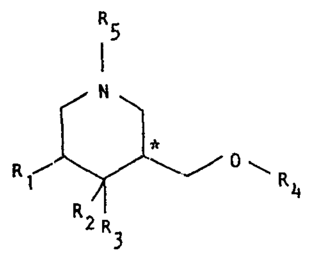

(I)

wherein

$R_1$ and $R_2$ are both hydrogen or together are a bond;

$R_3$ and $R_4$ are independently optionally substituted phenyl or naphthyl groups;

$R_5$ is a group $(CH_2)_nR_6$ wherein n is 1 or 2 and $R_6$ is an optionally substituted phenyl or naphthyl group having activity against disorders relating to impaired gastrointestinal motility, a process and intermediates for their preparation and their use as pharmaceuticals

EP 0 190 496 A2

Croydon Printing Company Ltd

ACTIVE COMPOUNDS

The present invention relates to novel compounds useful in the treatment and/or prophylaxis of disorders relating to the gastro-intestinal function, to a process for their preparation and to their use as pharmaceuticals.

U.S. Patent No. 3912743 discloses 3-substituted-1-alkyl-4-phenylpiperidine derivatives and U.S. Patent No. 4007196 discloses further derivatives including the compound, (-)-trans-4-(4'-fluorophenyl)-3-(3'4'-methylenedioxy-phenoxymethyl)piperidine, (commonly known as paroxetine), and processes by which they can be prepared. The compounds are described in the patents as inhibitors of 5-hydroxytryptamine uptake and, therefore, are of use in the treatment of depression. The patents also mention that the compounds are useful in the treatment of Parkinson's disease.

A group of novel piperidine derivatives has now been discovered and these compounds have activity against disorders relating to damaged gastro-intestinal tissue and to impaired gastro-intestinal motility. Examples of disorders relating to damaged gastro-intestinal tissue include peptic ulcers, such as gastric and duodenal ulcers. Examples of disorders relating to impaired gastro-intestinal motility include retarded gastric emptying, dyspepsia, flatulence, oesophagal reflux and peptic ulcer.

Accordingly, the present invention provides a compound of formula (I) or a pharmaceutically acceptable salt thereof:

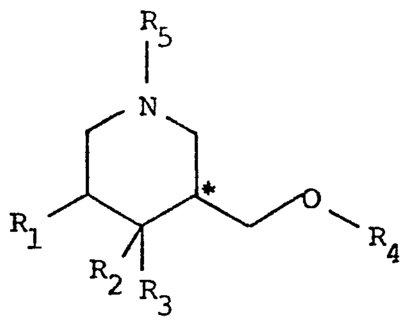

(I)

wherein:

$R_1$ and $R_2$ are both hydrogen or together are a bond;

$R_3$ and $R_4$ are independently optionally substituted phenyl or naphthyl groups;

$R_5$ is a group $(CH_2)_nR_6$ wherein n is 1 or 2 and $R_6$ is an optionally substituted phenyl or naphthyl group.

$R_1$ and $R_2$ are preferably both hydrogen.

Suitable optional phenyl or naphthyl substituents in $R_3$, $R_4$ and $R_6$ include one, two or three groups independently selected from halogen, $CF_3$, $C_{1-6}$ alkoxy, $C_{1-6}$ alkylthio, $C_{1-6}$ alkyl, nitro, cyano, carboxyl, hydroxy, $C_{1-6}$ alkoxycarbonyl, $C_{1-10}$ carboxylic acyl, and amino optionally substituted by one or two $C_{1-6}$ alkyl groups, disubstituted by $C_{3-6}$ polymethylene optionally containing oxygen, sulphur or $NR_7$ wherein $R_7$ is hydrogen or $C_{1-6}$ alkyl, or monosubstituted by $C_{1-4}$ alkanoyl; or $R_3$, $R_4$ and/or $R_6$ is/are disubstituted on adjacent carbon atoms by methylenedioxy, ethylenedioxy, $C_{3-5}$ polymethylene or $-CH=CH-(CH_2)_2-$.

Examples of the above substituents include fluoro, chloro, bromo, $CF_3$, methoxy, ethoxy, *n*- and *iso*-propoxy, methyl, ethyl, *n*- and *iso*-propyl, nitro, cyano, carboxyl, hydroxy, methoxycarbonyl, ethoxycarbonyl, *n*- and *iso*-propoxycarbonyl, formyl, acetyl, propionyl, amino optionally substituted by one or two methyl groups, disubstituted by $C_4$ or $C_5$ polymethylene or substituted by acetyl.

Favourably $R_3$ is phenyl monosubstituted by halogen, preferably 4-fluoro, or methoxy, such as 2-methoxy.

Favourably $R_4$ is phenyl 3,4 disubstituted by methylenedioxy, or $R_4$ is 2-methoxyphenyl.

Favourably $R_5$ is $CH_2R_6$ and $R_6$ is unsubstituted phenyl or phenyl monosubstituted by nitro or halo, preferably 4-substituted, in particular 4-fluorophenyl.

Pharmaceutically acceptable salts of the compounds of the formula (I) include acid addition salts with acids, such as the conventional pharmaceutically acceptable acids, for example hydrochloric, hydrobromic, maleic, phosphoric, acetic, fumaric, malonic, salicylic, citric, lactic, mandelic, tartaric and methanesulphonic; internal salts such as N-oxides; and quaternary ammonium salts with alkyl, phenylalkyl and cycloalkyl halides. Examples of quaternising agents include methyl, ethyl, *n*- and *iso*-propyl, benzyl, phenethyl chlorides, bromides and iodides.

The compounds of formula (I) have at least one asymmetric centre (indicated by '*' in formula I) and thus are capable of existing in a number of stereoisomeric forms. The invention extends to each of these isomeric forms and to mixtures thereof (including racemates). The different isomeric forms may be separated one from the other by conventional techniques or any given isomer may be obtained by a stereospecific synthesis.

The invention also provides a process for the preparation of a compound of formula (I) which process comprises reacting a compound of formula (II)

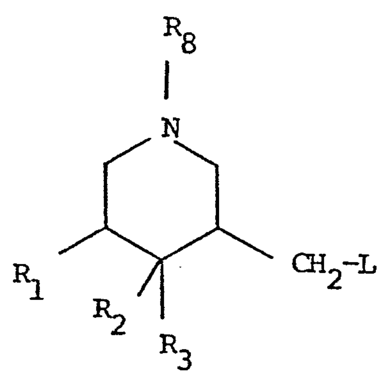

(II)

wherein:

L is a leaving group or $OR_4$;

$R_8$ is hydrogen when L is $OR_4$ or $(CH_2)_nR_6$ when L is a leaving group; and $R_1$, $R_2$ and $R_3$ are as defined in formula (I); with

i) $R_6(CH_2)_nQ$ wherein Q is a leaving group when $R_8$ is hydrogen); or

ii) $R_4$ OH or an alkali metal salt thereof (when L is a leaving group);

and thereafter optionally converting substituents in $R_3$, $R_4$ and/or $R_6$ to other substituents in $R_3$, $R_4$ and/or $R_6$ and/or forming a pharmaceutically acceptable salt.

The group Q in $R_6(CH_2)_nQ$ is a group readily displaceable by a nucleophile. Suitable values for Q include halogen, preferably chloro.

The reaction may take place under conventional conditions for nucleophilic displacements using amines. Suitable conditions include using an inert solvent such as dimethylformamide or acetone together with a base such as potassium carbonate at a temperature of 0 to 50°C, preferably at ambient temperature.

Preferably the alkali metal salt of $R_4OH$ is the sodium salt.

The group L in formula (II) when a leaving group is a group readily displaceable by a nucleophile.

Examples of such groups are hydroxy, halogen such as chloro and bromo or a sulphonate, such as methyl sulphonate, ethyl sulphonate or p-toluenesulphonate or benzenesulphonate.

If the leaving group is a halide or sulphonate, then the reaction is preferably carried out at a non-extreme temperature in an inert non-hydroxylic solvent, such as benzene, dimethylformamide, toluene or diethyl ether. It may also be carried out in the presence of an acid acceptor, such as an organic base, in particular a tertiary amine, such as triethylamine, trimethylamine,

pyridine or picoline, some of which can also function as the solvent. Alternatively, the acid acceptor can be inorganic, such as calcium carbonate, sodium carbonate or potassium carbonate.

The leaving group L may also be an activated phosphate formed by the reaction of diethylazidodicarboxylate and triphenyl phosphine with the corresponding compound wherein L is OH. The reaction preferably takes place in an inert solvent such as tetrahydrofuran at a temperature from ambient to reflux.

Alternatively the reaction may take place in the presence of a condensation promoting agent, such as dicyclohexylcarbodiimide, when L is hydroxy, optionally in the presence of a mineral acid or a metal ion, such as copper (II). The reaction may take place under conventional conditions.

The skilled man will appreciate that the choice or necessity of conversion of groups $R_3$, $R_4$ and/or $R_6$ to other groups $R_3$, $R_4$ and/or $R_6$ will be dictated by the nature and position of substituents on $R_3$, $R_4$ and $R_6$. It will be apparent that compounds of the formula (I) containing an $R_3$, $R_4$ or $R_6$ group which is convertible to another $R_3$, $R_4$ or $R_6$ group are useful novel intermediates. A number of such conversions is possible not only for the end compounds of formula (I) but also for their intermediates as follows:

(a) an hydrogen substituent is convertible to a nitro substituent by nitration;

(b) a nitro substituent is convertible to an amino substituent by reduction;

(c) a $C_{1-4}$ alkanoylamino substituent is convertible to an amino substituent by deacylation;

(d) an amino substituent is convertible to a $C_{1-4}$ alkanoylamino substituent by acylation; and

(e) a hydrogen substituent is convertible to a halogen substituent by halogenation.

Conversions (a) to (e), are only exemplary and are not exhaustive of the possibilities.

In reagard to (a), nitration is carried out in accordance with known procedures.

In regard to (b), the reduction is carried out with a reagent suitable for reducing nitroanisole to aminoanisole.

In regard to (c), deacylation is carried out by treatment with a base, such as an alkali metal hydroxide.

In regard to (d), the acylation is carried out with an acylating agent, such as the corresponding acid or acid chloride or acid anhydride. Formylation is carried out with the free acid or its mixed anhydride.

In regard to (e), halogenation is carried out with conventional halogenating agents.

The invention further provides novel intermediates within formula (II), of formula (III), and pharmaceutically acceptable salts thereof:

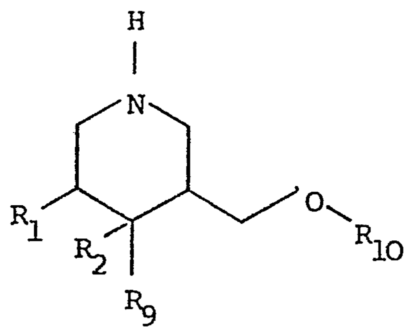

(III)

wherein:

$R_9$ is 3,4-methylenedioxyphenyl and $R_{10}$ is 4-fluorophenyl; or

$R_9$ is 4-fluorophenyl and $R_{10}$ is phenyl, 4-fluorophenyl, 2-methoxyphenyl, 4-methylphenyl, 3-trifluoromethylphenyl; or

$R_9$ is phenyl, 2-methoxyphenyl or 2-methylphenyl and $R_{10}$ is 3,4-methylenedioxyphenyl;

$R_1$ and $R_2$ are as defined in formula (I).

Intermediates of the formulae (II) and (III) may be prepared as described in the aforementioned U.S. Patents or by analogous methods thereto.

Alternatively, intermediates of formulae (II) and (III) may be prepared according to one of the following a) to d):

(Alk is a simple alkyl group such as ethyl).

a)

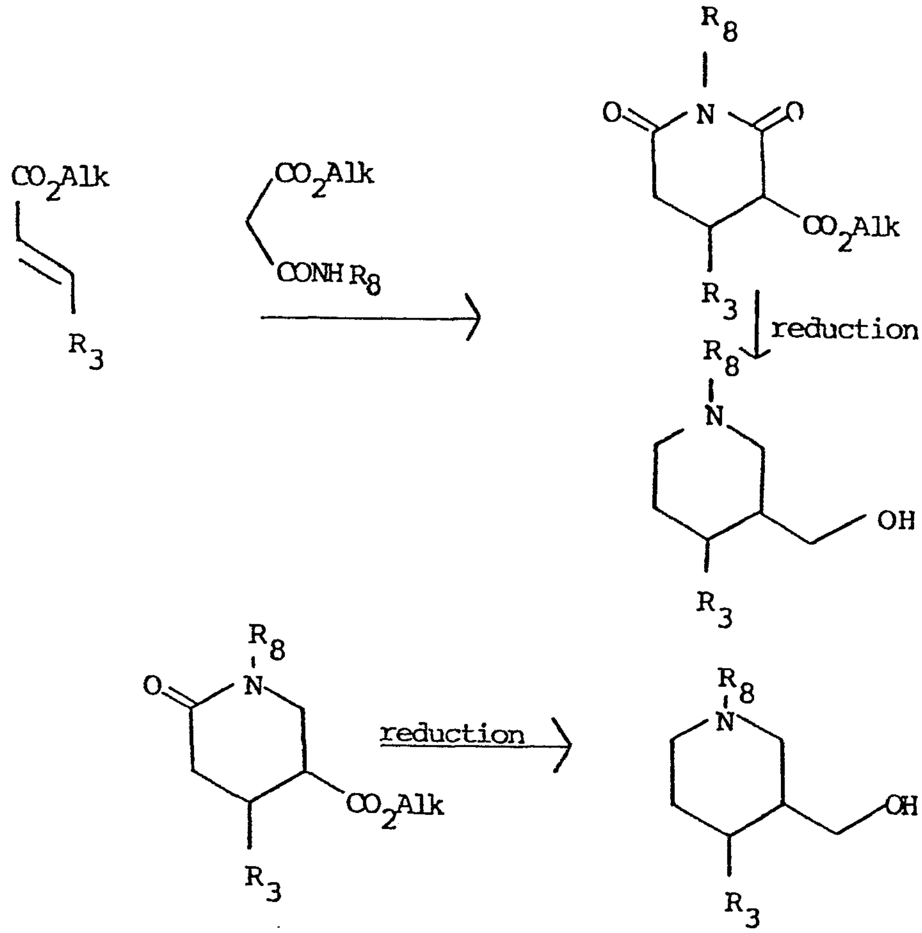


b)

W.H. Moos, R.D. Gless and H. Rapoport, J. Org. Chem., 1981, 46, 5064

c) via

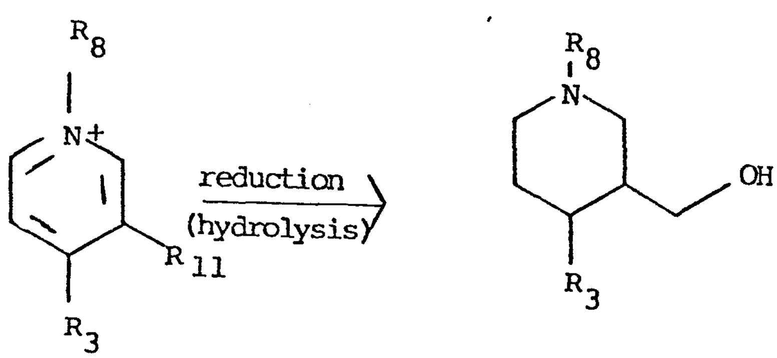

$R_{11}$ = $-CO_2Alk$

D.L. Comins, E.D. Strand and J.J. Herrick, Heterocycles, 1984, 22, 151

$R_{11}$

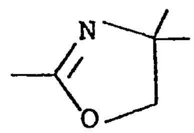

A.I. Meyers and R.A. Gabel, J. Org. Chem., 1982, 47, 2633 A.I. Meyers and N.R. Natele, Heterocycles, 1982, 18, 13. A.I. Meyers, N.R. Natele and D.G. Wettlanfer, Tetrahedron lett., 1981, 5723.

d)

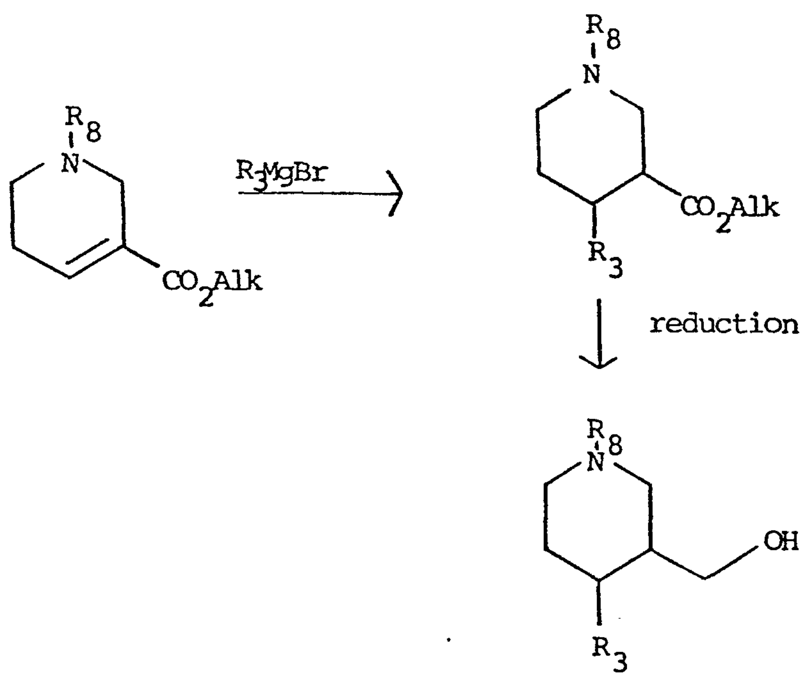


G. Lambrecht and E. Mutschlet, Arch. Pharm., 1975, 308, 676.

The invention also provides a pharmaceutical composition comprising a compound of formula (I) or a pharmaceutically acceptable salt thereof and a pharmaceutically acceptable carrier.

It is greatly preferred that the compound of formula (I) or a pharmaceutically acceptable salt thereof is administered in the form of a unit-dose composition, such as a unit-dose enteral, including oral, or parenteral composition.

Examples of oral compositions include tablets and capsules which generally contain conventional excipients, such as a binding agent, filler, lubricant, and disintegrating agent. An oral composition may also be in the form of a liquid, such as an aqueous or oily suspension, a solution, emulsion, syrup or elixir, or it may be in the form of a dry product for reconstitution with water or any other pharmaceutically acceptable liquid vehicle. Such liquid compositions generally contain conventional additives where appropriate, such as a suspending agent, emulsifying agent, preservative or flavouring agent.

Examples of parenteral compositions include suspensions and solutions which generally contain a surfactant or wetting agent and one or more adjuvants, such as a local anaesthetic, preservative or buffering agent. A parenteral solution may be prepared by dissolving the compound of formula (I) or a pharmaceutically acceptable salt thereof in an aqueous or non-aqueous vehicle and filter sterilizing it prior to filling into a vial or ampoule and sealing. A parenteral suspension may be prepared in much the same manner except that the compound of formula (I) or a pharmaceutically acceptable salt thereof is suspended, rather than dissolved, in the vehicle and that sterilization is carried out prior to suspension by exposure of the compound or salt to ethylene oxide.

Alternative composition types include suppositories.

A unit-dose composition, preferably, contains from 0.1 to 1000 mg, such as 0.5 to 500 mg of a compound of formula (I) or a pharmaceutically acceptable salt thereof. Such unit-dose compositions may be administered several times a day, such as one, two or three times a day such that the total daily dose is in the range mentioned hereinafter for effective treatment or prophylaxis.

The invention also provides a method for the treatment and/or prophylaxis of disorders relating to damaged gastro-intestinal tissue or to impaired gastro-intestinal motility in mammals, such as humans, which comprises the administration to the mammal of an effective amount of a compound of formula (I) or a pharmaceutically acceptable salt thereof.

The administration to the mammal may be by way of oral administration or parenteral administration.

An effective amount of a compound of formula (I) or a pharmaceutically acceptable salt thereof may be determined in accordance with the usual factors, such as the nature and severity of the disorder and the weight of the mammal requiring treatment. However, it is believed that an amount from 0.01 to 30 mg/kg per day should be sufficient for effective treatment or prophylaxis.

No toxicological effects are indicated at the aforementioned dosage range.

The invention also provides a compound of the formula (I) or a pharmaceutically acceptable salt thereof for use as an active therapeutic substance; and in particular for use in the treatment of disorders relating to damaged gastro-intestinal tissue and to impaired gastro-intestinal motility.

The following Examples illustrate the invention and the following descriptions illustrate the preparation of intermediates.

Description 1

(±) trans-3-Carbethoxy-4-(2'-methoxyphenyl)-piperidine-2,6-dione (D1) intermediate for compound (E2)

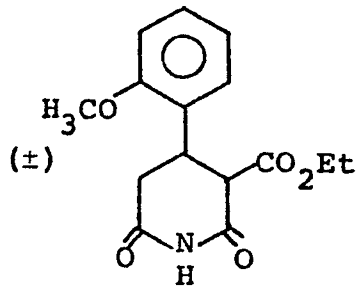

(D1)

To a stirred solution of ethyl malonamide (2.90g) in dry THF (80ml) was added potassium tert-butoxide (2.50g) followed by ethyl 2-methoxycinnamate (4.12g), and the whole left at R.T. for 36h. The whole was poured into water (200ml) and the product extracted into ethyl acetate (3 x 150ml), dried ($K_2CO_3$) and evaporated under reduced pressure. The crude product was purified by chromatography on silica gel ($CHCl_3$) to give D1 (2.23g, 38%) m.p. 112-13° (ethyl acetate - petroleum ether (40-60)).

nmr ($CDCl_3$, δ)

1.00 (t, 3H)
2.80-3.15 (m, 2H)
3.55-4.45 (m, 4H)
3.80 (s, 3H)
6.60-7.45 (m, 4H)
8.50-8.90 (br.s, 1H)

Description 2

(±) trans-3-Carbethoxy-4-(3',4'-methylenedioxyphenyl)-piperidine-2,6-dione (D2) intermediate for compound (E23)

(±)

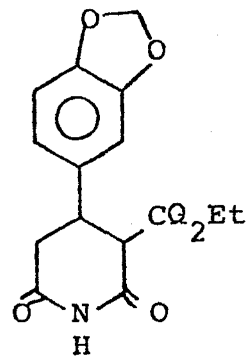

(D2)

To a stirred solution of ethyl malonamide (3.93g) in absolute ethanol (70ml) was added potassium tert-butoxide (3.36g) followed by ethyl-3,4-methylenedioxycinnamate (6.60g), and the whole heated at reflux for ¾hr. The near solid mass was allowed to cool, water (50ml) added and the product extracted into methylene chloride (3 x 70ml), dried ($K_2CO_3$) and evaporated under reduced pressure. Crystallisation from ether gave D2 (4.70g, 51%) as a white solid m.p. 139-40°C.

nmr ($CDCl_3$ + $D_6DMSO$, δ)

1.10 (t, 3H, J = 14Hz)
2.50-2.90 (m, 2H)
3.25-3.90 (m, 2H)
4.00 (q, 2H, J = 14Hz)
5.85 (s, 2H)
6.65 (s, 3H)

Description 3

(±) trans-3-Carbethoxy-4-phenylpiperidine-2,6-dione (D3) intermediate for compound (E15) and (E16)

(±)

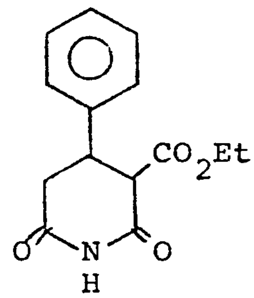

(D3)

To a stirred solution of ethyl malonamide (20.7g) in dry THF (500ml) was added potassium tert-butoxide (13.3g) followed by ethyl cinnamate (20g), and the whole left at R.T. for 4hr. The whole was poured into water (200ml) and the product extracted into ethyl acetate (3 x 150ml), dried ($K_2CO_3$) and evaporated under reduced pressure. The crude product was purified by chromatography on silica gel ($CHCl_3$) to give D3 (14.5g, 51%) m.p. 114-15$^o$ (ether) in two batches.

nmr ($CDCl_3$, $\delta$)

1.05 (t, J = 14Hz, 3H)
2.50-3.05 (m, 2H)
3.10-4.40 (m, 2H)
3.95 (q, J = 14Hz, 2H)
7.10 (br.s, 5H)
7.60-8.20 (br.s, 1H)

Description 4

(±) trans-4-(2'-methoxyphenyl)-3-piperidinemethanol (D4) intermediate for compound (E2)

(±) 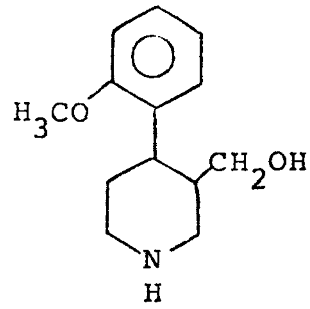

(D4)

To a stirred suspension of lithium aluminium hydride (3.30g) in dry THF (100ml), was added dropwise the imide D1 (12.65g) dissolved in dry THF (150ml) under an atmosphere of nitrogen, and the mixture heated under reflux for 6h. After being cooled, the reaction mixture was treated, sequentially, with water (3.30ml), 2.5N NaOH solution (5.00ml) and water (8.20ml). The solid was removed by filtration and the filtrate dried ($K_2CO_3$) and concentrated under reduced pressure to give D4 (8.00g, 83%) as an oil.

Description 5

(±) trans-4-(3',4'-Methylenedioxyphenyl)-3-piperidine methanol (D5) intermediate for compound (E23)

(±) 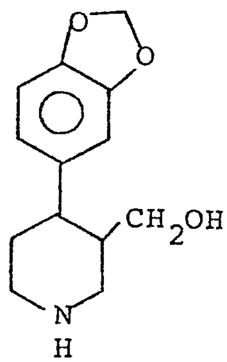

(D5)

To a stirred suspension of lithium aluminium hydride (1.3g) in dry THF (50ml), was added dropwise the imide D2 (4.56g) dissolved in dry THF (50ml) under an atmosphere of nitrogen, and the mixture heated under reflux for 4hr. After being cooled, the reaction mixture was treated, sequentially, with water (1.3ml), 2.5N NaOH solution (2.0ml) and water (3.2ml). The solid was removed by filtration and the filtrate dried ($K_2CO_3$) and concentrated under reduced pressure to give D5 (2.86g, 81%) as an oil.

nmr ($CDCl_3$, δ)

1.30-4.10 (m, 12H)

5.85 (s, 2H)

6.60 (s, 3H)

Description 6

(±) trans-4-phenyl-3-piperidinemethanol (D6)
intermediate for compound (E15) and (E16)

(±)

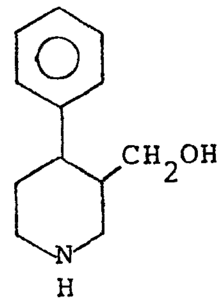

(D6)

Following the method outlined in description 5, D3 (10g) was converted to D6 (5.7g, 75%) as an oil.

Description 7

(±) trans-1-methoxycarbonyl-4-(2'-methoxyphenyl)-3-piperidinemethanol (D7) intermediate for compound (E2)

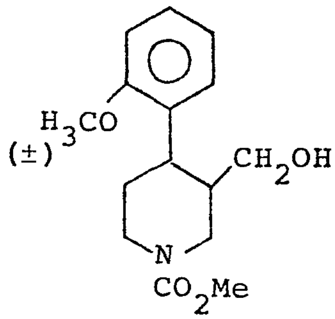

(D7)

To a stirred solution of the piperidinemethanol D4 (8.00g) in dry dichloromethane (100ml), containing triethylamine (5.00ml), at $0^o$ and under an atmosphere of nitrogen, was added dropwise methyl chloroformate (2.80ml) dissolved in dry dichloromethane (50ml). The whole was stirred at R.T. for 18h before 1N HCl (50ml) was added and the organic phase separated off. The aqueous phase was further extracted with dichloromethane (3 x 100ml), and the combined organic extracts dried ($K_2CO_3$) and evaporated under reduced pressure to give the carbamate D7 (8.28g, 82%) as an oil.

nmr ($CDCl_3$, δ)

1.40-4.60 (m, 11H)
3.68 (s, 3H)
3.75 (s, 3H)
6.60-7.50 (m, 4H)

Description 8

(±) trans-4-(3',4'-Methylenedioxyphenyl)-1-methoxycarbonyl-3-piperidinemethanol (D8) intermediate for compound (E23)

(±)

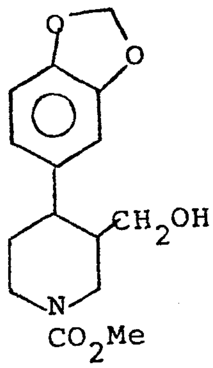

(D8)

To a stirred solution of the piperidinemethanol D5 (2.86g) in dry dichloromethane (50ml), containing triethylamine (1.66ml), at $0^{o}$ and under an atmosphere of nitrogen, was added dropwise, methyl chloroformate (0.94ml) dissolved in dry dichloromethane (30ml). The whole was stirred at R.T. for 4h before 1N HCl (20ml) was added and the organic phase separated off. The aqueous phase was further extracted with dichloromethane (3 x 50ml), and the combined organic extracts dried ($K_2CO_3$) and evaporated under reduced pressure to give the crude carbamate D8 (3.28g) which was purified by chromatography on silica gel (EtOAc) to give D8 (2.74g, 72%) as an oil.

nmr ($CDCl_3$, δ)

1.20-4.50 (m, 11H)
3.40 (s, 3H)
5.70 (s, 2H)
6.45 (s, 3H)

Description 9

(±) trans-1-methoxycarbonyl-4-phenyl-3-piperidinemethanol (D9) intermediate for compound (E15) and (E16)

(±)

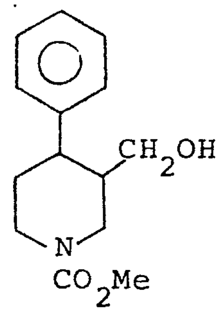

(D9)

Following the general method outlined in description 8, the piperidinemethanol D6 (5.0g) was converted to the carbamate D9 (3.7g, 59%) as an oil.

nmr ($CDCl_3$, $\delta$)

1.40-3.40 (m, 9H)
3.60 (s, 3H)
3.75-4.55 (m, 2H)
7.05 (s, 5H)

Description 10

(±) trans-1-Methoxycarbonyl-4-(2'-methoxyphenyl)-3-(3',4'-methylenedioxyphenoxymethyl)-piperidine (D10)

intermediate for compound (E2)

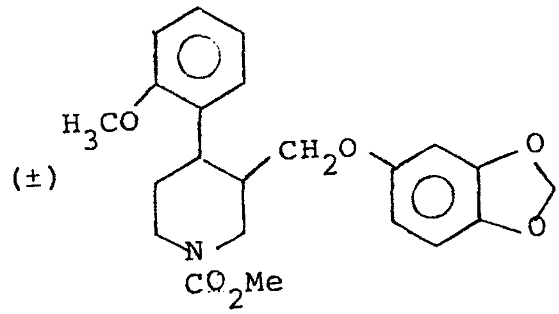

(D10)

To a stirred solution of the alcohol D7 (8.20g) in dry methylene chloride (80ml) containing triethylamine (4.00ml) at 0°, was added dropwise methanesulphonylchloride (3.37ml), dissolved in dry methylene chloride (30ml), over ca ¼h. The whole was then stirred at 0° for a further 1½h before being partitioned between water and dichloromethane. The organic phase was dried ($Na_2SO_4$), and the solvent was removed in vacuo. The mesylate (6.00g) thus produced was dissovled in dry DMF (50ml) and added to the sodium salt of 3,4-methylenedioxyphenol (2.63g), prepared by adding 80% sodium hydride (0.49g) to a stirred solution of the phenol (2.25g) in DMF (25ml) under an atmosphere of nitrogen. The whole was then heated to 90° for 3h before being allowed to cool to room temperature and poured into water (300ml). Recovery of the product into ether (3 x 100ml) gave, after drying ($K_2CO_3$) and evaporation under reduced pressure, crude D10 which was purified by chromatography on alumina (ether) to give the ether D10 (2.00g, 18%) as an oil.

nmr ($CDCl_3$, δ)

1.00-4.80 (m, 10H)
3.70 (s, 3H)
3.75 (s, 3H)
5.80 (s, 2H)
5.90-7.40 (m, 7H)

Description 11

(±) trans-3-(4'-Fluorophenoxymethyl)-1-methoxycarbonyl-4-(3',4'-methylenedioxyphenyl)-piperidine (D11)

intermediate for compound (E23)

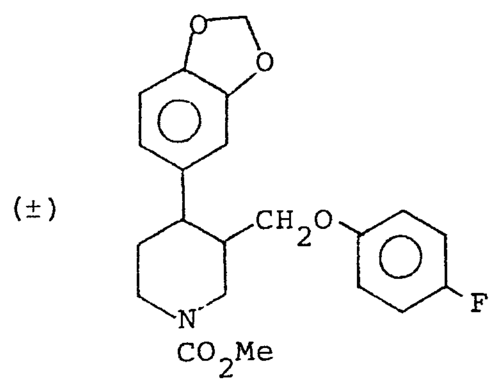

(D11)

To a stirred solution of the alcohol D8 (10.64g) in dry methylene chloride (200ml) containing triethylamine (5.6ml) at 0°, was added dropwise methanesulphonylchloride (3.08ml), dissolved in dry methylene chloride (30ml), over ca ¼hr. The whole was then stirred at 0° for a further 1½hr before being partitioned between water and dichloromethane. The organic phase was dried ($Na_2SO_4$), and the solvent was removed *in vacuo*. The mesylate (13.44g) thus produced was dissovled in dry DMF (50ml) and added to the sodium salt of 3,4-methylenedioxyphenol (6.2g), prepared by adding 80% sodium hydride (1.35g) to a stirred solution of the phenol (5.2g) in DMF (50ml) under an atmosphere of nitrogen. The whole was then heated to 90° for 3hr before being allowed to cool to room temperature and

poured into water (300ml). Recovery of the product into ether (3 x 100ml) gave, after drying ($K_2CO_3$) and evaporation under reduced pressure, crude D11 which was purified by chromatography on silica gel ($CH_3Cl$) to give the ether D11 (6.92g, 54%) as a solid m.p. 122-23$^o$ (ether).

nmr ($CDCl_3$, $\delta$)

1.50-2.25 (m, 3H)
2.30-3.10 (m, 3H)
3.25-3.90 (m, 2H)
3.73 (s, 3H)
5.91 (s, 2H)
6.50-7.30 (m, 7H)

Description 12

(±) trans-1-Methoxycarbonyl-3-(3',4'-methylenedioxy-phenoxymethyl)-4-phenylpiperidine (D12) intermediate for compound (E15) and (E16)

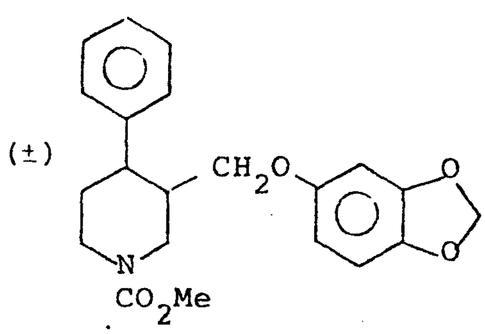

(D12)

Following the general method outlined in description 11, the alcohol D9 (3.70g) was converted to the ether D12 (4.71g, 94%) as an oil.

nmr ($CDCl_3$, δ)

1.00-4.60 (m, 10H)
3.60 (s, 3H)
5.75 (s, 2H)
5.85-6.65 (m, 3H)
7.05 (br.s, 5H)

Description 13

(-) trans-4-(4'-Fluorophenyl)-1-methyl-3-(2'-methoxyphenoxymethyl)-piperidine (D13) intermediate for compound (E3)

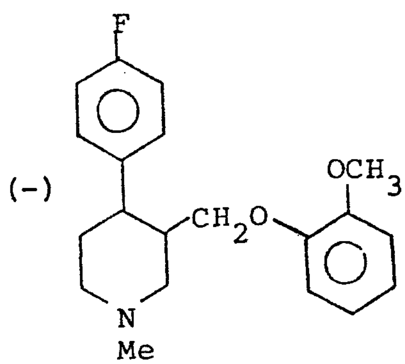

(D13)

Following the general method outlined in description 10, except that heating in DMF was prolonged to 18h, (-) trans-4-(4'fluorophenyl)-1-methyl-3-piperidine-methanol (11.15g) was converted to the ether D13 (7.24g, 47%) as an oil (hydrochloride salt m.p. 158° (acetone-ether)).

nmr ($CDCl_3$, δ)

1.50-4.25 (m, 10H)
2.85 (s, 3H)
3.80 (s, 3H)
6.35-7.50 (m, 8H)

Description 14

(-) trans-4-(4'Fluorophenyl)-1-methyl-3-(3'-trifluoromethylphenoxymethyl)-piperidine (D14) intermediate for compound (E24)

(-)

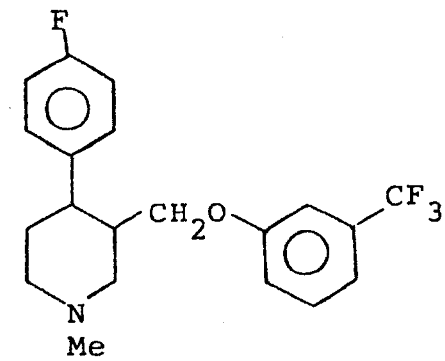

(D14)

Following the general method outlined in description 10, except that phenylsulphonylchloride was substituted for methanesulphonylchloride, (-) trans-4-(4'-fluorophenyl)-1-methyl-3-piperidinemethanol (9.31g) was converted to the ether D14 (11.20g, 72%) as an oil (hydrochloride salt m.p. 145-47°C (acetone-ether)).

nmr ($D_2O$, δ, HCl salt)

1.50-4.10 (m, 10H)

2.85 (s, 3H)

6.25-7.35 (m, 8H)

Description 15

(-) trans-4-(4'-Fluorophenyl)-1-methoxycarbonyl-3-(2'-methoxyphenoxymethyl)-piperidine (D15) intermediate for compound (E3)

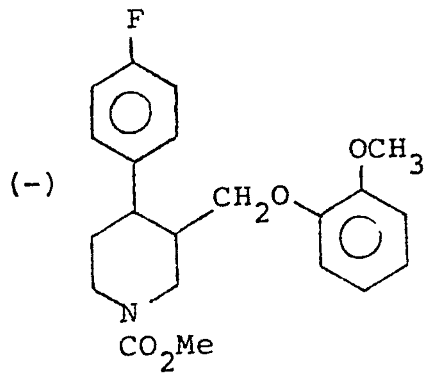

(D15)

To a stirred solution of the amine D13 (4.19g) in dry toluene, under an atmosphere of nitrogen, was added dropwise methyl chloroformate (11ml). The whole was heated under reflux for 24h, before being cooled and ethyl acetate (100ml) added. The organic phase was washed with 5N HCl (50ml), 10% NaOH (50ml) and water (100ml), dried ($Na_2SO_4$) and evaporated under reduced pressure to give the carbamate D15 (3.1g, 65%) as an oil.

nmr ($CDCl_3$, $\delta$)

1.10-4.80 (m, 10H)
3.67 (s, 3H)
3.80 (s, 3H)
6.30-7.50 (m, 8H)

Description 16

(-) trans-4-(4'-Fluorophenyl)-1-methoxycarbonyl-3-(3'-trifluoromethylphenoxymethyl)-piperidine (D16)

intermediate for compound (E24)

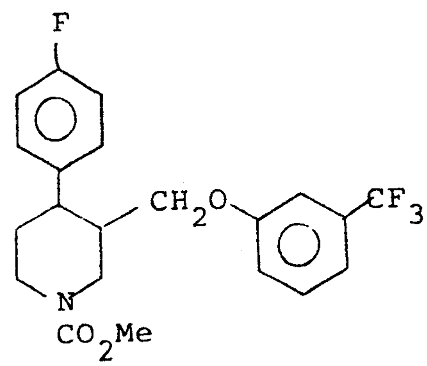

(D16)

Following the general method outlined in description 15, the amine D14 (9.60g) was converted to the carbamate D16 (9.60, 89%) as an oil.

nmr ($CDCl_3$, δ)

1.45-4.75 (m, 10H)

3.65 (s, 3H)

6.62-7.65 (m, 8H)

Description 17

(±)-4-(4'-Fluorophenyl)-3-(3',4'-methylenedioxyphenoxymethyl)-1-methyl-1,2,3,6-tetrahydropyridine (D17) intermediate for compounds (E7), (E8), (E11), (E12), and (E13)

(±)

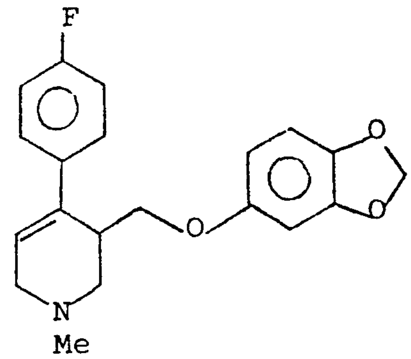

(D17)

Benzene sulphonyl chloride (107.3ml) was added to a solution of (-)-4-(4'-fluorophenyl)-3-hydroxymethyl-1-methyl-1,2,3,6-tetrahydropyridine (157.0g) in dichloromethane (750ml) at 0° and in the presence of triethylamine (155.0ml) over 1h. The mixture was then stirred and allowed to warm to RT over a further 1½h. After washing with water (100ml), the organic solution was evaporated under reduced pressure and the residue dissolved in toluene (750ml). To this solution was added a solution of 3,4-methylenedioxyphenol (98.3g) in methyl isobutylcarbinol (600ml) and a solution of sodium hydroxide (28.9g) in water (60ml). The mixture was then stirred and heated under reflux (94°) for 6h before being cooled to RT and washed with water (100ml). The organic solution was evaporated to dryness under reduced pressure and the residue crystallised from ethanol to give the title compound D17 (170g) m.p. 97-98.5°.

nmr ($CDCl_3$, δ)

2.38 (s, 3H)
2.48 (dd, 1H)
2.87 (ddd, 1H)
3.06 (dd, 1H)
3.13 (m, 1H)
3.31 (dd, 1H)
3.68 (dd, 1H)
3.96 (dd, 1H)
5.86 (s, 2H)
5.99 (dd, 1H)
6.22 (dd, 1H)
6.42 (d, 1H)
6.63 (d, 1H)
7.01 (dd, J = 9 Hz, 2H)
7.34 (dd, J = 5.5 Hz, 2H)

Description 18

(±)-cis-4-(4'-Fluorophenyl)-3-(3',4'-methylenedioxyphenoxymethyl)-1-methylpiperidine (D18) intermediate for compounds (E7), (E8), (E12), and (E13)

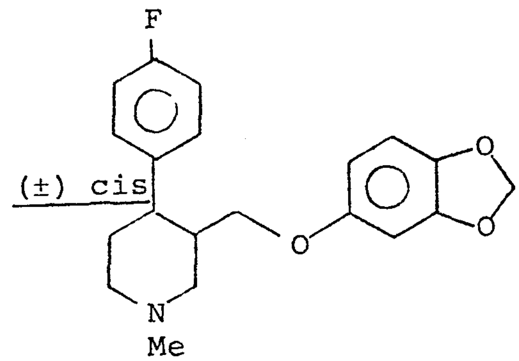

(D18)

The tetrahydropyridine D17 (168.0g) was reduced using hydrogen with a palladium on charcoal catalyst and ethanol (500ml) as solvent. The reaction was carried out at 50$^{o}$ and 25psi hydrogen pressure. On completion of the reaction (20h) the catalyst was removed by filtration and the resultant solution was evaporated to dryness *in vacuo*. The residue was crystallised from ethanol to give the title compound D18 (115g) as a colourless crystalline solid. mp 110-111$^{o}$.

nmr ($CDCl_3$, δ)

1.77 (m, 1H)
2.00 (m, 1H)
2.08 (d, 1H)
2.19 (dd, 1H)
2.30 (s, 3H)
2.36 (m, 1H)
2.90 (ddd, 1H)
3.05 (ddd, 1H)
3.18 (m, 1H)
3.46 (dd, 1H)
4.17 (dd, 1H)

5.86 (s, 2H)
6.12 (dd, 1H)
6.30 (d, 1H)
6.59 (d, 1H)
7.00 (dd, J = 9 Hz, 2H)
7.18 (dd, J = 5.5 Hz, 2H)

Description 19

(±)-cis-4-(4'-Fluorophenyl)-3-(3',4'-methylenedioxyphenoxymethyl)-1-phenoxycarbonylpiperidine (D19)

intermediate for compounds (E7), (E8), (E12) and (E13)

(±) cis

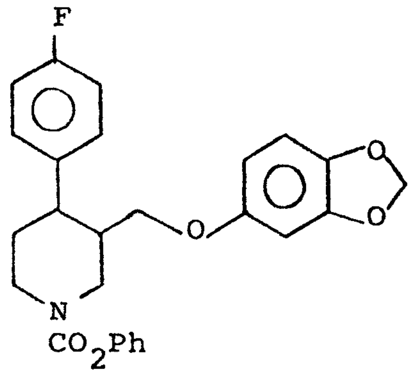

(D19)

Phenylchloroformate (53.5ml) in dry toluene (105ml) was added to a solution of D18 (115g) in dry toluene (600ml) over 1h, maintaining the temperature at ca. $0^{o}$. The mixture was then stirred for 5h at RT. After the addition of dichloromethane to dissolve the precipitated solid, the organic phase was successively washed with 1N hydrochloric acid (50ml), water (3 x 50ml), 1N sodium hydroxide solution (50ml), and water (50ml). The organic solution was then evaporated to dryness *in vacuo* and the residue crystallised from ethanol to give the title compound D19 (129g) as a colourless crystalline solid mp. 111.5-113.5$^{o}$.

nmr ($CDCl_3$, $\delta$)

1.83 (m, 1H)
2.08 (dddd, 1H)
2.39 (m, 1H)
2.98 (m, 1H)
3.17 (ddd, 1H)
3.26 (m, 1H)
3.57 (dd, 1H)
3.86 (dd, 1H)
4.53 (m, 1H)
4.77 (m, 1H)
5.85 (m, 2H)
6.13 (dd, 1H)
6.31 (d, 1H)
6.50 (d, 1H)
6.70-7.40 (m, 9H)

Description 20

(±) 1-Ethoxycarbonyl-3-methoxycarbonyl-4-(2'-methylphenyl)-1,4-dihydropyridine (D20) intermediate for compound (E17)

(±)

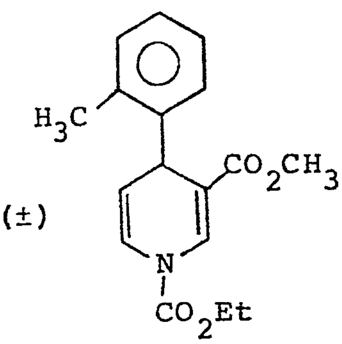

(D20)

2-Methylphenyl magnesium bromide was prepared by the addition of 2-bromotoluene (21.3g) to magnesium (2.75g) in dry THF (100ml) under nitrogen atmosphere.

To a stirred suspension of cupric chloride (400mg) in dry THF (250ml) under nitrogen atomosphere was added ethyl chloroformate (12.28g). The mixture was cooled to $0^oC$, and a solution of methyl nicotinate (15.53g) in dry THF (50ml) added over 5 minutes. After stirring for a further 10 minutes at $0^oC$, the previously prepared Grignard reagent was added dropwise. Following this addition, the reaction mixture was stirred for a further 20 minutes.

The mixture was diluted with ethyl acetate (750ml), and a mixture of $NH_4OH:NH_4Cl$ (sat$^d$. aq.) (1:1, 250ml) added. This two phase system was stirred vigorously for 10-15 minutes. The organic phase was then separated, and washed with $NH_4OH:NH_4Cl$ (1:1, 2 x 75ml), 2M HCl (4 x 75ml) and brine (250ml), then dried ($Na_2SO_4$) and evaporated to give the title compound D20 as a light red-brown oil which solidified upon standing (28.1g). This material was used without further purification.

n.m.r. ($CDCl_3$, δ)
1.40 (t, 3H)
2.40 (s, 3H)
3.60 (s, 3H)
4.30 (q, 2H)
4.60-4.80 (m, 1H)
4.90-5.20 (m, 1H)
6.60-6.90 (m, 1H)
7.00-7.50 (m, 4H)
8.20 (d, 1H)
($^1H$ NMR shows the presence of approximately 10% of the 6-aryl isomer).

Description 21

3-Methoxycarbonyl-4-(2'-methylphenyl)pyridine (D21) intermediate for compound (E17)

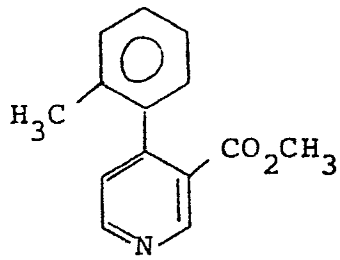

(D21)

Crude dihydropyridine D20 (28.1g) was dissolved in warm decalin (75ml) and sulphur (3g) added. The mixture was heated under reflux under an atmosphere of nitrogen for 16hr, then diluted with ethyl acetate (300ml) and extracted with 2M HCl (4 x 25ml). The combined aqueous extracts were washed with ethyl acetate (100ml), then basified with 20% sodium hydroxide solution in the presence of dichloromethane (100ml). The aqueous phase was extracted with dichloromethane (3 x 100ml), and the combined organic extracts were dried ($K_2CO_3$) and evaporated under reduced pressure to give the title compound D21 as an oil (11.38g) which was used without further purification.

nmr ($CDCl_3$, δ)

2.10 (3, 3H)
3.60 (3, 3H)
6.80-7.40 (m, 5H)
8.80 (d, 1H)
9.20 (s, 1H)

($^1$H-NMR shows the presence of approximately 10% of the 6-aryl isomer).

Description 22

3-Methoxycarbonyl-1-methyl-4-(2'-methylphenyl)-pyridinium bromide (D22) intermediate for compound (E17)

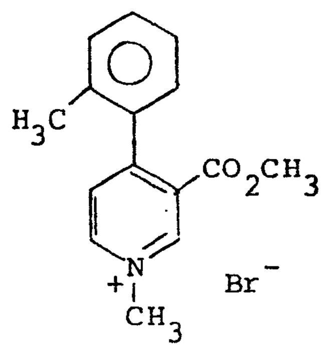

(D22)

Crude aryl-pyridine D21 (6.36g) was dissolved in acetone (15ml) and the solution cooled to 0°C. Methylbromide (6ml) was added, the reaction vessel sealed and the whole stirred for 16hr at room temperature. After cooling to 0°C the resulting yellow solid was collected by filtration, washed with acetone and dried *in vacuo*, to give the title compound D22 (6.67g) free from the 6-aryl impurity.

nmr ($CDCl_3$, δ)

2.10 (s, 3H)
3.70 (s, 3H)
4.90 (s, 3H)
6.80-7.40 (m, 4H)
7.70-8.00 (m, 1H)
9.70-10.10 (m, 2H)

Description 23

(±)-cis-3-Methoxycarbonyl-1-methyl-4-(2'-methylphenyl) piperidine (D23) intermediate for compound (E17)

(±) cis

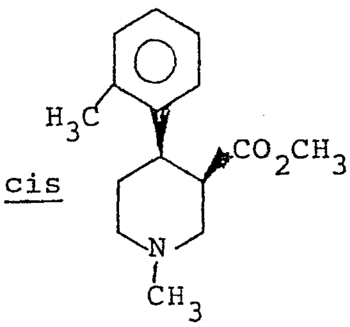

(D23)

3-Methoxycarbonyl-1-methyl-4-(2'-methylphenyl) pyridinium bromide D22 (6.67g) was dissolved in ethanol (100ml) and hydrogenated (24hr, 1 atm., 45°C) in the presence of $PtO_2$ (400mg) catalyst.

The catalyst was removed by filtration through Kieselguhr. Water was added to the filtrate and the solution made alkaline (pH 8-9) with potassium carbonate solution. The solvent was evaporated *in vacuo*, and the residue partitioned between water (30ml) and dichloromethane (30ml). The aqueous phase was further extracted with dichloromethane (3 x 30ml). The combined organic extracts were dried ($K_2CO_3$) and evaporated *in vacuo* to give the title compound D23 (5.03g, 98%) as a pale oil which solidified on standing.

nmr ($CDCl_3$, δ)

1.30-3.50 (m, 8H)
2.26 (s, 3H)
2.30 (s, 3H)
3.40 (s, 3H)
6.80-7.50 (m, 4H)

Description 24

(±)-trans-Methoxycarbonyl-1-methyl-4-(2'-methylphenyl) piperidine (D24) intermediate for compound (E17)

(±) trans

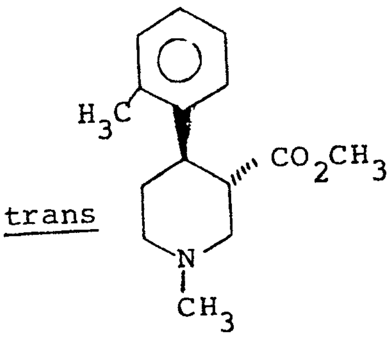

(D24)

The (±)-cis piperidyl ester D23 (5.00g) dissolved in dry toluene (25ml), was added to a solution of sodium methoxide (200mg, 0.25 eq) in dry toluene (20ml) under an atomosphere of nitrogen. The mixture was heated at 55°C for 3hr. After cooling to RT, water (10ml) was added, and the organic phase was separated, and the aqueous phase extracted with ethyl acetate (2 x 20ml). The combined organic phase was dried ($Na_2SO_4$) and the solvent evaporated in vacuo to give the title compound D24 as a white solid (4.20g).

nmr ($CDCl_3$, δ)

1.40-2.60 (m, 4H)
2.30 (s, 6H)
2.70-3.40 (m, 4H)
3.35 (s, 3H)
6.90-7.30 (m, 4H)

Description 25

(±)-trans-1-Methyl-4-(2'-methylphenyl)-3-piperidinemethanol (D25) intermediate for compound (E17)

(±) trans

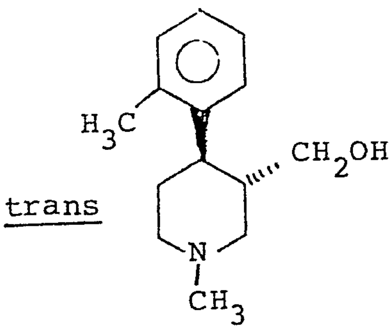

(D25)

Following the general method outlined in description 4, the piperidylester D24 (3.7g) was converted to the title compound D25 (3.21g, 98%). m.p. 129-30$^{o}$ (ethyl acetate/60-80 petroleum ether).

nmr ($CDCl_3$, $\delta$)

1.46-3.59 (m, 17H)

6.83-7.30 (m, 4H)

Description 26

(±)-trans-3-(3',4'-Methylenedioxyphenoxymethyl)-1-methyl-4-(2'-methylphenyl)-piperidine (D26)
intermediate for compound (E17)

(±) trans

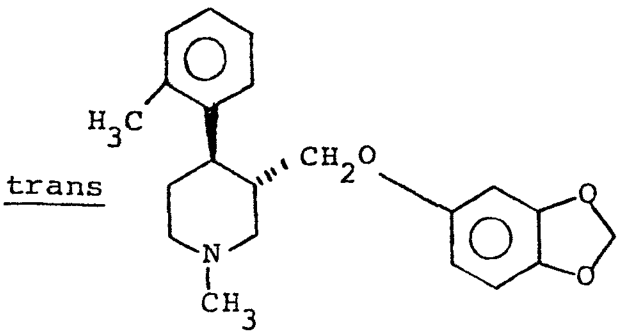

(D26)

Following the general method outlined in description 10, except that phenylsulphonylchloride was substituted for methanesulphonylchloride and that the phenylsulphonate thus produced was then heated to 45°C for 4hrs, (±)-trans-1-methyl-4-(2'-methylphenyl)-3-piperidinemethanol D25 (0.875g) was converted to the ether D26 (0.77g, 57%) as an oil.

n.m.r. ($CDCl_2$, δ)

1.50-3.60 (m, 10H)
2.27 (s, 3H)
2.31 (s, 3H)
5.80 (s, 2H)
6.00 (d.d., 1H)
6.30 (d, 1H)
6.50 (d, 1H)
6.90-7.40 (m, 4H)

Description 27

(±) trans-4-(4'-Fluorophenyl)-1-methyl-3-(4'-methylphenoxymethyl)piperidine (D27) intermediate for compounds (E18) and (E19)

(±) trans

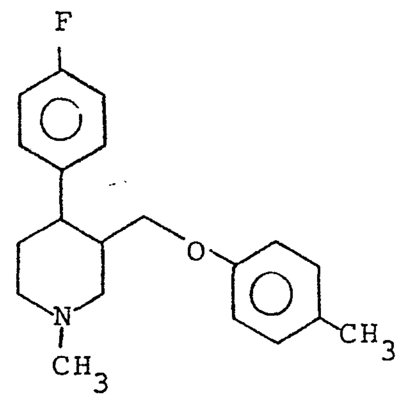

(D27)

Following the general method outlined in description 14, except that 4-methylphenol was substituted for 3,4-methylenedioxyphenol, (±) trans-4-(4'-fluorophenyl)-1-methyl-3-piperidinemethanol (2.40g) was converted to the ether D27 (2.95g, 87%) as an oil.
nmr ($CDCl_3$, δ)

1.50-3.60 (m, 10H)
2.20 (s, 3H)
2.30 (s, 3H)
6.40-7.30 (m, 8H)

Description 28

(±) trans-4-(4'-Fluorophenyl)-1-methyl-3-phenoxymethyl-piperidine (D28) intermediate for compound (E20)

(±) trans

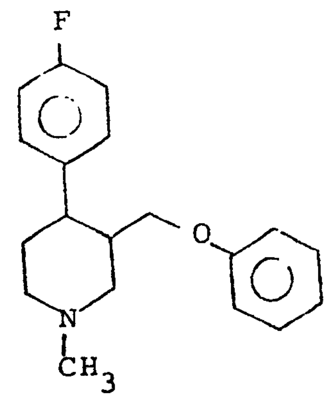

(D28)

Following the general method outlined in description 14, except that phenol was substituted for 3,4-methylenedioxyphenol, (±) trans-4-(4'-fluorophenyl)-1-methyl-3-piperidinemethanol (2.40g) was converted to the ether D28 (1.95g, 60%) as an oil.

nmr ($CDCl_3$, δ)

1.60-3.60 (m, 10H)

2.30 (s, 3H)

6.50-7.50 (m, 9H)

Description 29

(±) trans-3-(4'-Fluorophenoxymethyl)-4-(4'-fluorophenyl)-1-methylpiperidine (D28) intermediate for compounds (E21) and (E22)

(±) trans

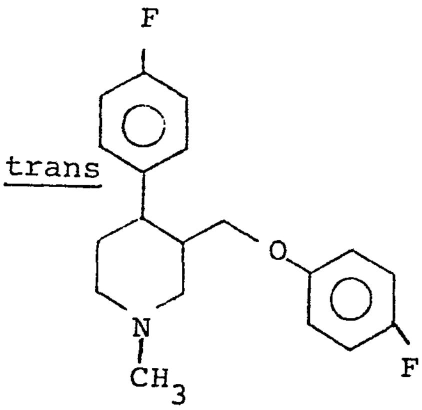

(D29)

Following the general method outlined in description 14, except that 4-fluorophenol was substituted for 3,4-methylenedioxyphenol, (±) trans-4-(4'-fluorophenyl)-1-methyl-3-piperidinemethanol (2.58g) was converted to the ether D29 (2.93g, 80%) as an oil.

nmr ($CDCl_3$, δ)

1.60-3.70 (m, 10H)

2.30 (s, 3H)

6.40-7.30 (m, 8H)

Description 30

(±) trans-4-(2'-Methoxyphenyl)-3-(3',4'-methylenedioxy-phenoxymethyl)-piperidine hydrochloride (D30)

intermediate for compound (E2)

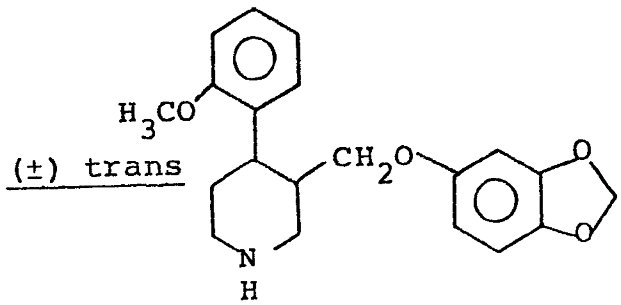

(±) trans

.HCl

(D30)

To a solution of the carbamate D10 (2.36g) in dry dichloromethane (50ml) was added trimethylsilyl iodide (2.00ml), and the whole heated under reflux under an atmosphere of nitrogen for 1h. After cooling to room temperature the solvent was removed under reduced pressure, replaced by fresh dry dichloromethane (20ml) and methanol (5ml) carefully added. After stirring for ½h, water (20ml) was added and the product extracted into dichloromethane (3 x 50ml), dried ($K_2CO_3$) and evaporated under reduced pressure to give a crude product. This crude product was dissolved in ether (50ml), neutral alumina (Brockmann grade I) (6g) added, and the whole stirred for 1h. The alumina was removed by filtration, washed with ether (20ml) and the organic phase concentrated under reduced pressure to give the free base of D30 (1.70g, 84%) as an oil. This oil was converted to the hydrochloride salt D30 (0.80g, 36%) m.p. 164° (ethanol-ether).

nmr ($CDCl_3$, δ, free base)

1.40-3.70 (m, 11H)

3.75 (s, 3H)

5.80 (s, 2H)

5.85-7.35 (m, 7H)

Description 31

(±) trans-3-(4'-Fluorophenoxymethyl)-4-(3',4'-methylenedioxyphenyl)-piperidine maleate (D31)

intermediate for compound (E23)

(±) trans

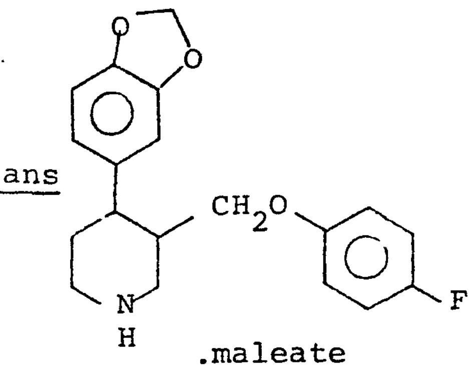

.maleate

(D31)

Following the general method outlined in description 30, the carbamate D11 (4.90g) was converted to the title compound D31 (1.17g, 20%) m.p. 144.5-46°C (methanol-ether).

nmr ($D_6$DMSO, δ)

1.50-4.10 (m, 10H)
5.98 (s, 2H)
6.10 (s, 2H)
6.50-7.50 (m, 7H)
8.00-9.25 (m, 1H)

Description 32

(±) trans-3-(3',4'-Methylenedioxyphenoxymethyl)-4-phenylpiperidine tartarate (D32) intermediate for compounds (E15) and (E16)

(±) trans

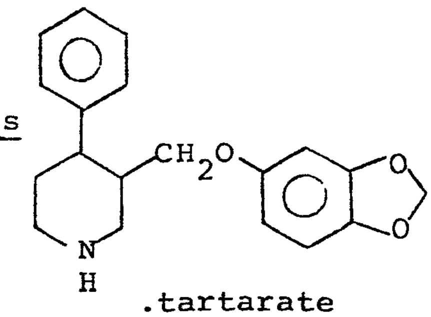

.tartarate

(D32)

Following the general method outlined in description 30, the carbamate D12 (3.75g) was converted to the tartrate salt D32 (0.30g, 7%) m.p. 163-65$^{o}$ (ethanol).

nmr ($CDCl_3$, δ, free base)

0.65-3.85 (m, 11H)

5.55 (s, 2H)

5.65-6.40 (m, 3H)

6.90 (br.s, 5H)

Description 33

<u>(-) trans-4-(4'-Fluorophenyl)-3-(2'-methoxyphenoxy-methyl)-piperidine maleate (D33)</u> intermediate for compound (E3)

(-) trans

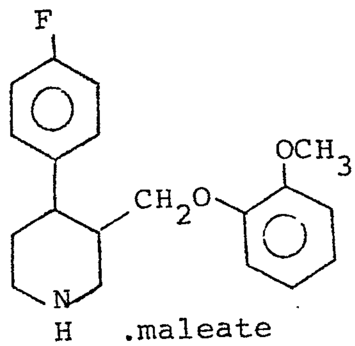

.maleate

(D33)

Following the general method outlined in description 30, the carbamate D15 (3.10g) was converted to the maleate salt D33 (1.30g, 36%) m.p. 135-38°C (methanol-ether).

nmr ($CDCl_3$, δ, free base)

1.40-3.80 (m, 11H)

3.90 (s, 3H)

6.30-7.40 (m, 8H)

Description 34

(-) trans-4-(4'-Fluorophenyl)-3-(3'-trifluoromethylphenoxymethyl)piperidine tartarate (D34) intermediate for compound (E24)

(-) trans

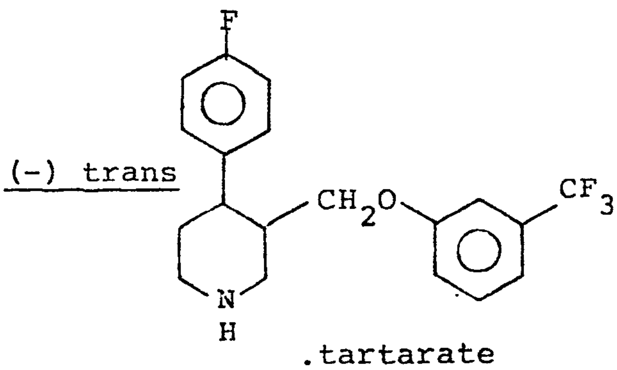

.tartarate

(D34)

Following the general method outlined in description 30, the carbamate D16 (9.60g) was converted to the tartrate salt D34 (1.70g, 15%) m.p. 144-48°C (ethanol).
nmr ($CDCl_3$, δ, free base)

1.50-3.90 (m, 11H)

6.70-7.55 (m, 8H)

Description 35

(±)-4-(4'-Fluorophenyl)-3-(3',4'-methylenedioxyphenoxymethyl)-1,2,3,6-tetrahydropyridine hydrochloride (D35) intermediate for compound (E11)

(±)

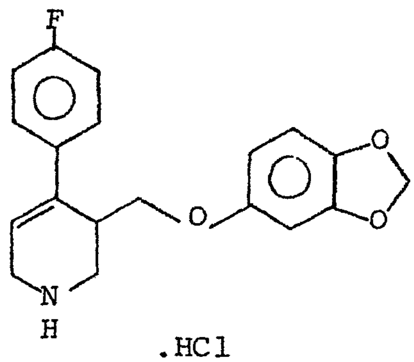

.HCl

(D35)

To a solution of α-chloroethylchloroformate (0.25g) in dry methylene chloride (0.2ml) at 0°, was added dropwide the amine D17 (0.5g), dissolved in dry methylene chloride (1.25ml), over a period of ¼h. The whole was then stirred at R.T. for 41h. The solution was then cooled to 0°, methanol (20ml) added, and the whole heated under reflux for 1h. After cooling to R.T. the solvent was removed *in vacuo* and the crude product partitioned between ethyl acetate and aqueous potassium carbonate. The organic phase was dried ($Na_2SO_4$) and evaporated *in vacuo* to give crude free base D35 which was purified by chromatography on alumina (ethyl acetate/ether) to give the free base of D35 (0.43g, 90%) as an oil. This oil was converted to the hydrochloride salt D35 (0.38g, 71%) m.p. 120° (ethanol-ether).

nmr ($D_6$ DMSO, δ)

3.15-3.60 (m, 3H)
3.65-3.88 (m, 3H)
3.88-4.10 (m, 1H)
5.94 (s, 2H)
6.05-6.10 (m, 1H)
6.25 (dd, 1H)
6.56 (d, 1H)
6.76 (d, 1H)
7.13-7.30 (m, 2H)
7.40-7.55 (m, 2H)
8.60-9.90 (br.s, 1H)

Descriptions 36 and 37

(+) and (-)-cis-4-(4'-Fluorophenyl)-3-(3',4'-methylenedioxyphenoxymethyl)piperidine hydrochloride (D36) and (D37) intermediates for compounds (E7), (E8), (E12) and (E13)

(+) cis

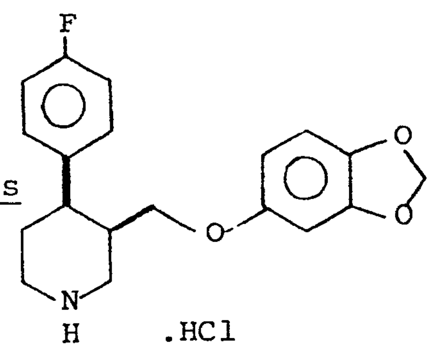

(D36)

(-) cis

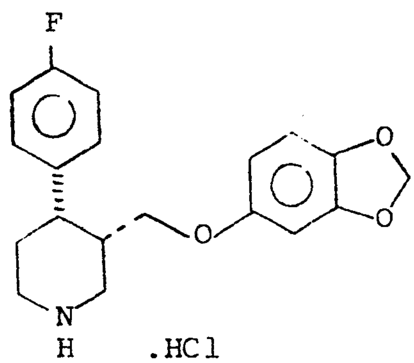

(D37)

The carbamate (D19) (129.0g) was dissolved in 2-methoxyethanol (300ml) with heating to ca. 60°. Potassium hydroxide (60.5g) was added over 1h then the mixture was heated to reflux at 122° for 2½h. After cooling the mixture, water (300ml) was added and the aqueous phase was extracted with toluene (3 x 150ml). The organic phase was then evaporated to dryness *in*

vacuo to give the crude racemic base (D36) and (D37) (96.0g)

52g of this material was dissolved in methanol (500ml) and was added to a methanolic solution (500ml) of D(-)-tartaric acid (23.8g). The D(-)-tartrate of the (+)-cis isomer (18.8g) thus obtained was dissolved in ethanol (100ml) and concentrated hydrochloric acid (3.7ml) added to give the title compound D36 (10.7g) as a colourless crystalline solid $[\alpha]^{26} = +118.3^{o}$ (C = 1 in methanol).

The methanol solution remaining after the formation of the (+)-cis D(-)-tartrate was evaporated to dryness in vacuo and the residue converted back to the free base. This was dissolved in methanol and added to a solution of L(+)-tartaric acid (21.0g) in methanol (500ml). The L(+)-tartrate of the (-)-cis-isomer (21.0g) thus obtained was dissolved in ethanol (100ml) and concentrated hydrochloric acid (4.4ml) added to give the title compound D37 (11.4g) as a colourless crystalline solid $[\alpha]^{26} = -115.4^{o}$ (C = 1 in methanol).

nmr ($D_6$ DMSO, δ)

1.88 (m, 1H)
2.22 (m, 1H)
2.55 (m, 1H)
3.05 (m, 1H)
3.38 (m, 5H)
4.26 (dd, 1H)
5.93 (s, 2H)
6.21 (dd, 1H)
6.52 (d, 1H)
6.74 (d, 1H)
7.17 (dd, J = 9 Hz, 2H)
7.33 (dd, J = 5.5 Hz, 2H)
9.35 (s, br, 2H)

Description 38

(±)-trans-3-(3',4'-Methylenedioxyphenoxymethyl)-4-(2'-methylphenyl)-piperidine hydrochloride (D38)

intermediate for compound (E17)

(±) trans

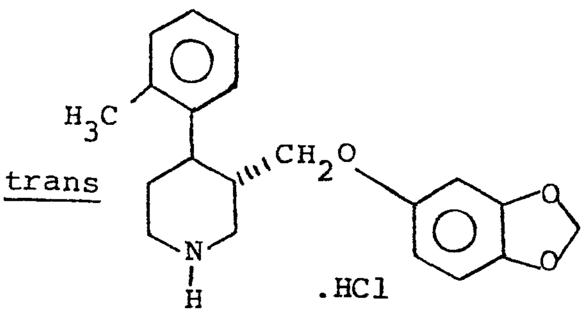

(D38)

The ether D26 (0.77g) was dissolved in dry toluene (5ml), and diluted with pentane (5ml). The cloudy solution was filtered through Kieselguhr, and the residue washed a little toluene-pentane (1:1). The combined filtrate was evaporated to dryness *in vacuo*. The residue was then dissolved in dry toluene (5ml) and the solution cooled to 0°C, under nigrogen. α-Chloroethyl chloroformate (0.275ml) was added with stirring and a white precipitate formed immediately. The reaction was allowed to warm to room temperature and then stirred at this temperature for 18hr. The small amount of remaining solid was removed by filtration through Kieselguhr. The filtrate was concentrated *in vacuo* (ca. 1ml) and then methanol (5ml) was added. The solution was allowed to stand at room temperature for 20hr and then evaporated to dryness *in vacuo* to give the title compound D38 (0.49g, 60%) as a white solid.

0190496

Description 39

(±) trans-4-(4'-Fluorophenyl)-3-(4'-methylphenoxymethyl)-piperidine hydrochloride (D39) intermediate for compounds (E18) and (E19)

(±) trans

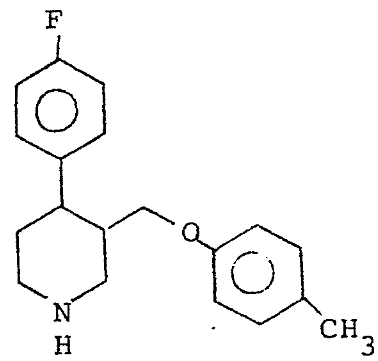

.HCl

(D39)

Following the general method outlined in description 38, (±)-trans-4-(4'-fluorophenyl)-1-methyl-3-(4'-methylphenoxymethyl)piperidine (2.95g) was converted to the piperidine hydrochloride D39 (2.07g, 65%) as a foam.

Description 40

(±)-trans-4-(4'-Fluorophenyl)-3-phenoxymethyl-piperidine-hydrochloride (D40) intermediate for compound (E20)

(±) trans

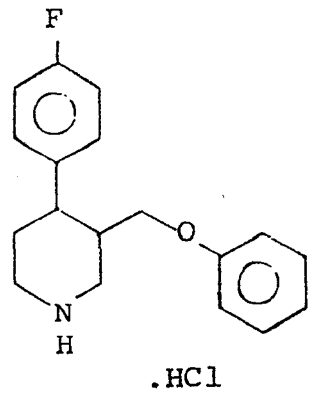

(D40)

Following the general method outlined in description 38, (±)-trans-4-(4'-fluorophenyl)-1-methyl-3-phenoxymethylpiperidine (1.95g) was converted to the piperidine hydrochloride D40 (2.11g, 84%) as a foam. m.s. Observed mass = 285.1523, theoretical mass = 285.1529 for $C_{18}H_{20}NOF$.

Description 41

(±)-trans-3-(4'-Fluorophenoxymethyl)-4-(4'-fluorophenyl)piperidine hydrochloride (D41) intermediate for compounds (E21) and (E22)

(±) trans

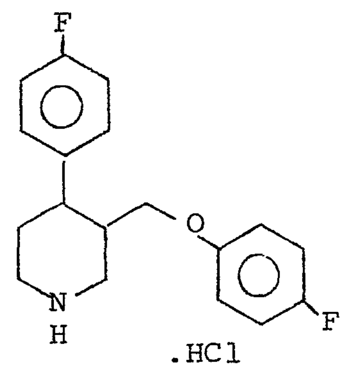

(D41)

Following the general method outlined in description 38, (±)-trans-3-(4'-fluorophenoxymethyl)-4-(4'-fluorophenyl)-1-methylpiperidine D29 (2.91g) was converted to the piperidine hydrochloride D41 (2.41g, 77%) as a foam.
m.s. Observed mass = 303.1443, theoretical mass = 303.1434 for $C_{18}H_{19}NOF_2$.

Example 1

(-)-trans-1-Benzyl-4-(4'-fluorophenyl)-3-(3',4'-methylenedioxyphenoxymethyl)piperidine hydrochloride (E1)

(-) trans

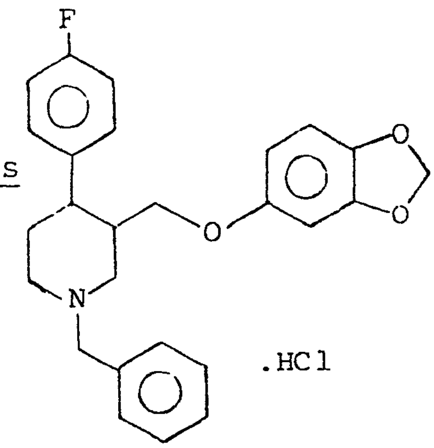

(E1)

Potassium carbonate (3.45g) was added to a stirred solution of (-)-trans-4-(4'-fluorophenyl)-3-(3',4'-methylenedioxyphenoxy methyl)piperidine (4.11g) in dimethylformamide (74ml). Benzyl chloride (1.58g) was then added dropwise over five minutes to the stirred suspension at ambient temperature. The reaction mixture was then stirred at ambient temperature for 18h. The inorganic solid was removed by filtration and washed with dichloromethane (50ml). Water (60ml) was added to the filtrate and then 40% sodium hydroxide solution was added until the pH was 14. The dichloromethane layer was separated off and the aqueous layer was washed with a further 40ml of dichloromethane. The dichloromethane extracts were combined and dried over sodium sulphate, filtered and evaporated under reduced pressure to yield (-)-trans-1-benzyl-4-(4'-fluorophenyl)-3-(3',4'-methylenedioxyphenoxymethyl)piperidine as a white gum (4.05g, 77%).

The (-)-<u>trans</u>-1-benzyl-4-(4'-fluorophenyl)-3-(3',4'-methylenedioxyphenoxymethyl) piperidine was dissolved in ethanol (12ml) and conc. hydrochloric acid (2.4ml) was added slowly with stirring. The hydrochloride salt immediately crystallised out and was filtered off and recrystallised from ethanol (20ml) to give the title compound E1 as white crystals (3.7g, 84%), m.p. 239°C.

nmr ($D_6DMSO$, $\delta$)

1.91 (d, 1H)
2.36 (dd, 1H)
2.82 (m, 2H)
3.03 (m, 2H)
3.3-3.5 (complex, 2H + $H_2O$ from solvent)
3.59 (d, 2H)
4.39 (Br.s, 2H)
5.93 (s, 2H)
6.19 (dd, 1H)
6.47 (d, 1H)
6.75 (d, 1H)
7.1-7.3 (complex, 4H)
7.46 (m, 3H)
7.70 (m, 2H)
11.66 (Br.s, 1H)

Example 2

(±)trans 1-Benzyl-4-(2'-methoxyphenyl)-3-(3',4'-methylenedioxyphenoxymethyl)piperidine hydrochloride (E2)

(±) trans

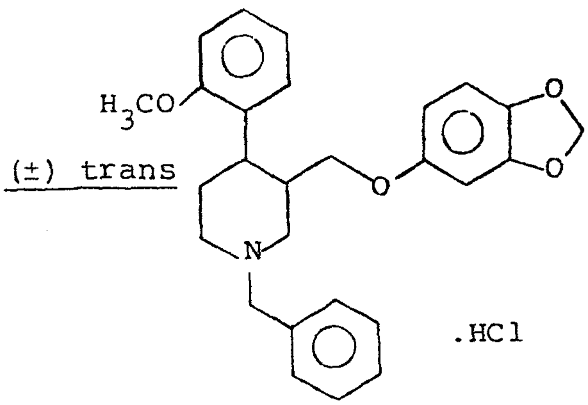

(E2)

Following the general method outlined in Example 1, the amine D30 (0.29g) was converted to the title compound E2 (0.27g, 75%) m.p. 221-22° (ethanol-ether).

nmr ($D_6$DMSO, δ)

1.70-1.95 (m, 1H)
2.00-2.30 (m, 1H)
2.60-3.20 (m, 4H)
3.25-3.85 (m, 4H)
3.35 (s, 3H)
4.25-4.50 (m, 2H)
5.92 (s, 2H)
6.14 (dd, 1H)
6.40 (d, 1H)
6.75 (d, 1H)
6.90-7.35 (m, 4H)
7.40-7.75 (m, 5H)

Example 3

(-)trans-1-Benzyl-4-(4'-fluorophenyl)-3-(2'-methoxyphenoxymethyl)piperidine fumarate (E3)

(-) trans

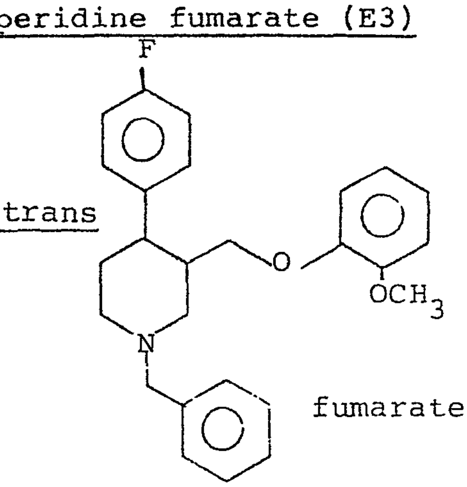

fumarate

(E3)

Following the general method outlined in Example 1, the amine D33 (1.00g) was converted to the title compound E3 (0.40g, 25%) m.p. 142-44° (ethanol-ether).

nmr ($CDCl_3$, δ, free base)

1.70-2.65 (m, 6H)
2.97-3.20 (m, 1H)
3.30-3.90 (m, 5H)
3.78 (s, 3H)
6.50-6.70 (m, 1H)
6.70-7.05 (m, 5H)
7.10-7.50 (m, 7H)

Example 4

(-)trans-4-(4'-Fluorophenyl)-3-(3',4'-methylenedioxyphenoxymethyl)-1-(4'-nitrobenzyl)piperidine hydrochloride (E4)

(-) trans

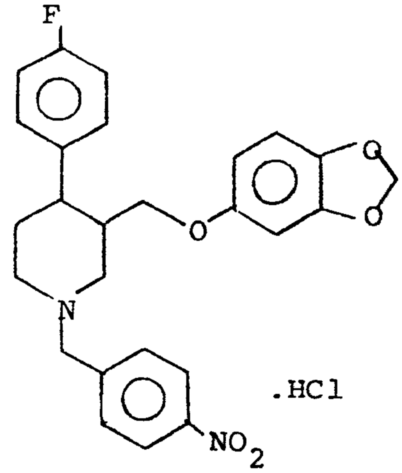

(E4)

Following the general method outlined in Example 1, (-)trans-4-(4'-fluorophenyl)-3-(3',4'-methylenedioxyphenoxymethyl)piperidine hydrochloride (1.00g) was converted to the title compound E4 (0.27g, 20%) m.p. 250° (ethanol-ether).

nmr ($D_6$DMSO, δ)

1.80-2.05 (m, 1H)
2.07-2.38 (m, 1H)
2.60-3.72 (m, 8H)
4.40-4.70 (m, 2H)
5.93 (s, 2H)
6.19 (dd, 1H)
6.47 (d, 1H)
6.74 (d, 1H)
7.06-7.35 (m, 4H)
7.95 (d, 2H)
8.33 (d, 2H)

Example 5

(+)trans-1-Benzyl-4-(4'-fluorophenyl)-3-(3',4'-methylenedioxyphenoxymethyl)piperidine hydrochloride (E5)

(+) trans

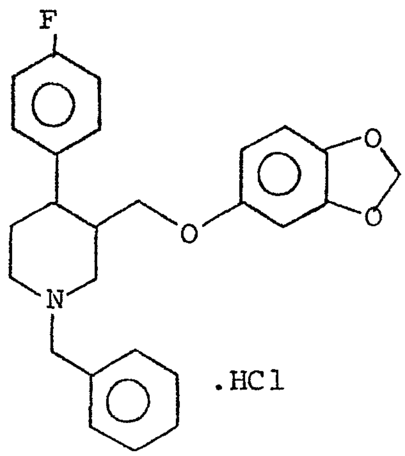

.HCl

Following the general method outlined in Example 1 (+)-trans-4-(4'fluorophenyl)-3-(3',4'-methylenedioxyphenoxymethyl)piperidine hydrochloride (1.00g) was converted to the title compound E5 (0.88g, 70%) m.p. 235° (ethanol-ether).

nmr ($D_6$DMSO, δ)

1.80-2.00 (m, 1H)
2.16-2.45 (m, 1H)
2.60-3.20 (m, 4H)
3.20-3.70 (m, 4H)
4.25-4.50 (m, 2H)
5.95 (s, 2H)
6.19 (dd, 1H)
6.47 (d, 1H)
6.75 (d, 1H)
7.05-7.30 (m, 4H)
7.48 (m, 3H)
7.60-7.80 (m, 2H)

Example 6

(-)-trans-4-(4'-Fluorophenyl)-3-(3',4'-methylenedioxyphenoxymethyl-1-((2'-ethyl)phenyl)piperidine hydrochloride (E6)

(-) trans

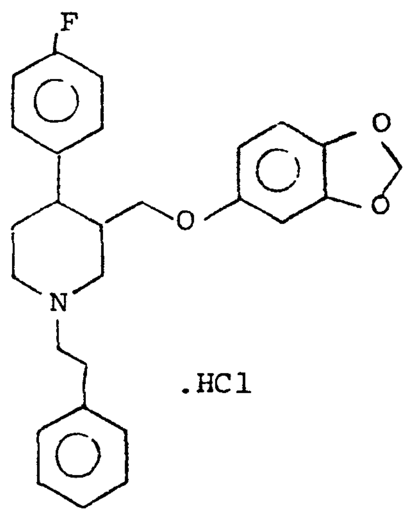

(E6)

Following the general method outlined in Example 1, (-)-trans-4-(4'-fluorophenyl)-3-(3',4'-methylenedioxyphenoxymethyl)piperidine hydrochloride (2.00g) was converted to the title compound E6 (1.00g, 36%) m.p. 211-13$^{o}$ (ethanol-ether).

nmr ($D_6$DMSO, δ)

1.90-2.06 (m, 1H)
2.10-2.40 (m, 1H)
2.58-3.96 (m, 12H)
5.95 (s, 2H)
6.24 (dd, 1H)
6.52 (d, 1H)
6.76 (d, 1H)
7.04-7.53 (m, 9H)

Example 7

(+)cis-1-Benzyl-4-(4'-fluorophenyl)3-(3',4'-methylene-dioxyphenoxymethyl)piperidine hydrochloride (E7)

(+) cis

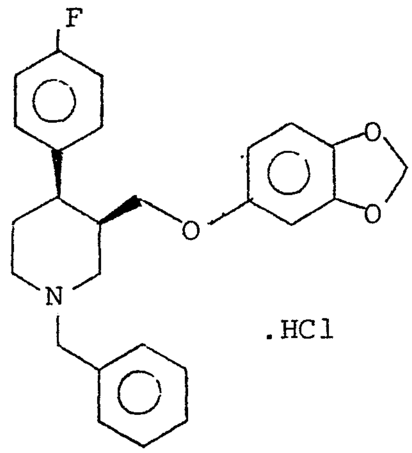

.HCl

(E7)

Following the general method outlined in Example 1, the amine D36 (0.40g) was converted to the title compound E7 (0.25g, 50%) m.p. 110-14° (ethanol-ether)

nmr ($D_6DMSO$, δ)

1.85-2.10 (m, 1H)
2.30-2.50 (m, 1H)
2.55-2.73 (m, 1H)
2.80-3.79 (m, 6H)
4.15-4.60 (m, 3H)
5.92 (s, 2H)
6.09 (dd, 1H)
6.34 (d, 1H)
6.72 (d, 1H)
7.02-7.80 (m, 9H)

Example 8

(-)cis-1-Benzyl-4-(4'-fluorophenyl)-3-(3',4'-methylenedioxyphenoxymethyl)piperidine hydrochloride (E8)

(-) cis

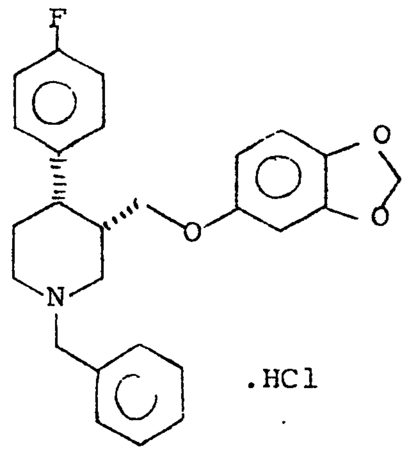

.HCl

(E8)

Following the general method outlined in Example 1, the amine D37 (0.40g) was converted to the title compound E8 (0.22g, 45%) m.p. 110-14° (ethanol-ether)
nmr ($D_6$DMSO, δ)

1.88-2.10 (m, 1H)
2.25-2.75 (m, 2H)
3.00-3.75 (m, 6H)
4.20-4.60 (m, 3H)
5.91 (s, 2H)
6.08 (dd, 1H)
6.35 (d, 1H)
6.73 (d, 1H)
7.09-7.80 (m, 9H)

Example 9

(-) trans-1-(4'-fluorobenzyl)-4-(4'-fluorophenyl)-3-(3',4'-methylenedioxyphenoxymethyl)piperidine hydrochloride (E9)

(-) trans

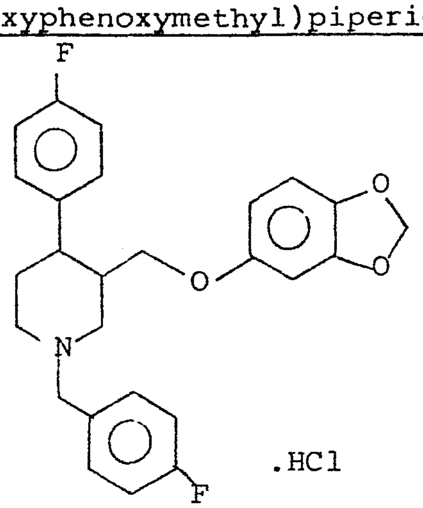

.HCl

(E9)

To a stirred solution of (-) trans-4-(4'-fluorophenyl)-3-(3',4'-methylenedioxyphenoxymethyl)piperidine hydrochloride (2.00g) in DMF (20ml) was added potassium carbonate (2.30g) and 4-fluorobenzyl chloride (0.87g, 0.72ml). The whole was then stirred at R.T. for 18h before being poured into water (100ml) and extracted into ether (3 x 80ml). The combined organic extracts were dried ($Na_2SO_4$) and evaporated under reduced pressure to give the crude free base of E9 (2.43g) as a white solid m.p. 104.5-5° (ethanol). This solid was converted to its hydrochloride salt E9 (1.28g, 50%) m.p. 210-12° (ethanol-ether)

nmr ($D_6$DMSO, δ)

| | |
|---|---|
| 1.80-2.03 (m, 1H) | 6.48 (d, 1H) |
| 2.15-2.46 (m, 1H) | 6.75 (d, 1H) |
| 2.60-3.20 (m, 4H) | 7.07-7.45 (m, 6H) |
| 3.23-3.75 (m, 4H) | 7.60-7.90 (m, 2H) |
| 4.41 (br.s, 2H) | |
| 5.94 (s, 2H) | |
| 6.20 (dd, 1H) | |

Example 10

(-)-trans-1-(4'-chlorobenzyl)-4-(4'-fluorophenyl)-3-(3',4'-methylenedioxyphenoxymethyl)piperidine hydrochloride (E10)

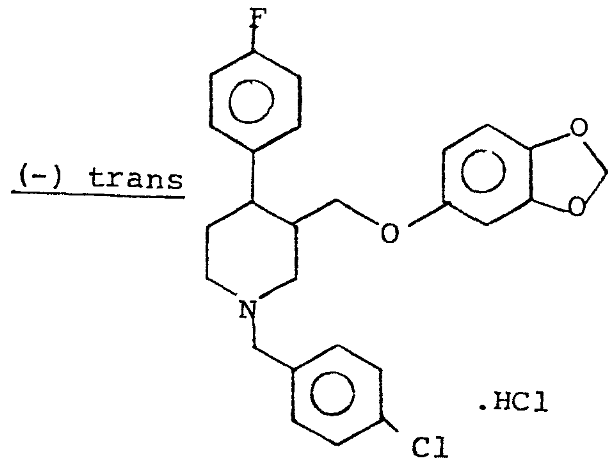

(E10)

Following the method outlined in Example 9, (-)-trans-4-(4'-fluorophenyl)-3-(3',4'-methylenedioxyphenoxymethyl)piperidine hydrochloride (2.00g) was converted to the title compound E10 (1.26g, 47%) m.p. 228-31$^{o}$ (ethanol-ether).

nmr ($D_6$DMSO, δ)

1.80-2.00 (m, 1H)
2.23-2.50 (m, 1H)
2.70-3.20 (m, 4H)
3.30-3.70 (m, 4H)
4.20-4.50 (m, 2H)
5.95 (s, 2H)
6.20 (dd, 1H)
6.49 (d, 1H)
6.74 (d, 1H)
7.06-7.32 (m, 4H)
7.55 (d, 2H)
7.75 (d, 2H)

Example 11

(±)-1-Benzyl-4-(4'-fluorophenyl)-3-(3,4-methylenedioxyphenoxymethyl)-1,2,3,6-tetrahydropyridine hydrochloride

(E11)

(±)

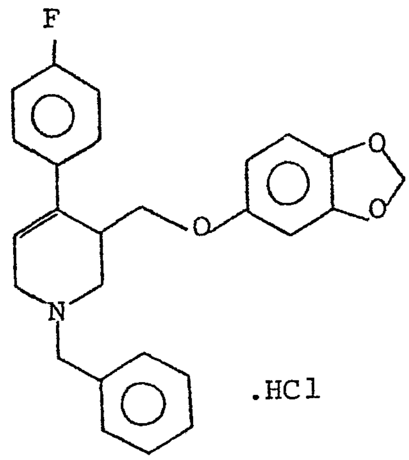

.HCl

(E11)

Following the general method outlined in Example 9, the amine D35 (7.20g) was converted to the title compound E11 (4.30g, 43%) m.p. 122-25° (ethanol-ether).

nmr ($D_6$ DMSO, δ)

3.20-3.48 (m, 1H)
3.48-4.10 (m, 5½H)
4.20-4.70 (m, 2½H)
5.80-5.90 (m, ½H)
5.90-6.00 (m, 2H)
6.05-6.30 (m, 1½H)
6.38-6.50 (m, 1H)
6.73 (d, 1H)
7.10-7.60 (m, 7H)
7.60-7.86 (m, 2H)
10.86,11.50 (2 x br.s, 1H)

Example 12

(+) cis-1-(4'-Fluorobenzyl)-4-(4'-fluorophenyl)-3-(3',4'-methylenedioxyphenoxymethyl)piperidine hydrochloride (E12)

(+) cis

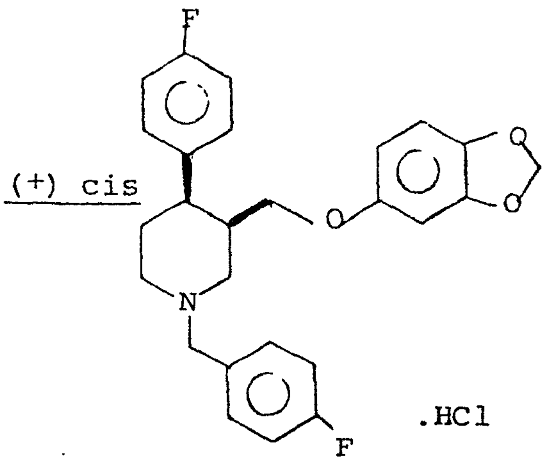

.HCl

(E12)

Following the general method outlined in Example 9, the amine D36 (2.00g) was converted to the title compound E12 (1.24g, 48%) m.p. 214-19$^{o}$ (ethanol-ether).

nmr ($D_6$ DMSO, $\delta$)

1.85-2.10 (m, 1H)
2.33-2.88 (m, 2H)
2.90-3.75 (m, 6H)
4.16-4.68 (m, 3H)
5.95 (s, 2H)
6.12, 6.17 (2 x dd, 1H)
6.38, 6.48 (2 x d, 1H)
6.70, 6.74 (2 x d, 1H)
7.08-7.40, 7.50-7.65 (2 x m, 6H)
7.77-8.03 (m, 2H)
11.08, 11.66 (2 x br.s, 1H)

Example 13

(-) cis-1-(4'-Fluorobenzyl)-4-(4'-fluorophenyl)-3-(3',4'-methylenedioxyphenoxymethyl)piperidine hydrochloride (E13)

(-) cis

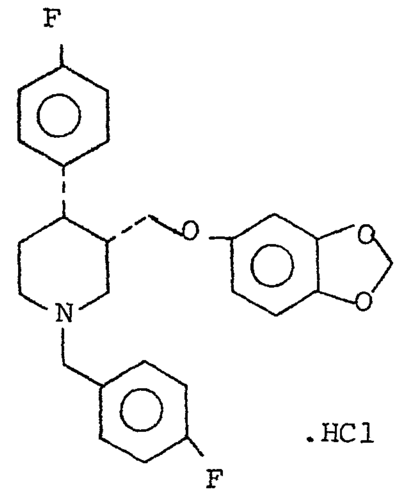

.HCl

(E13)

Following the general method outlined in Example 9, the amine D37 (1.00g) was converted to the title compound E13 (0.80g, 62%) m.p. 208-13° (ethanol-ether).

nmr ($D_6$DMSO, δ)

1.94-2.12 (m, 1H)
2.50-2.82 (m, 2H)
3.05-3.85 (m, 6H)
4.20-4.39 (m, 1H)
4.39-4.60 (m, 2H)
5.88,5.90 (2 x s, 2H)
6.10 (dd, 1H)
6.28,6.30 (2 x d, 1H)
6.59,6.61 (2 x d, 1H)
6.97-7.32,7.40-7.54 (2 x m, 6H)
7.70-7.97 (m, 2H)
10.82,11.58 (2 x br.s, 1H)

Example 14

(-) trans-1-(2'-Fluorobenzyl)-4-(4'-fluorophenyl)-3-(3',4-methylenedioxyphenoxymethyl)piperidine hydrochloride (E14)

(-) trans

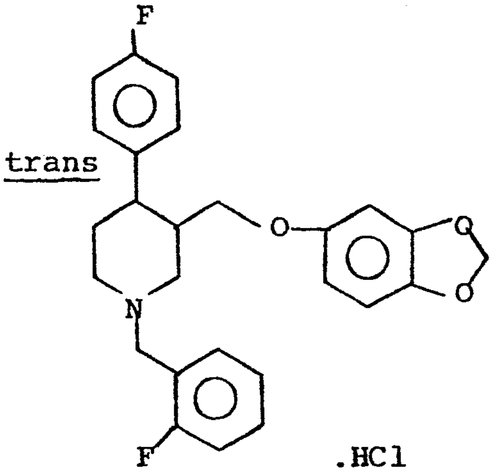

.HCl

(E14)

Following the general method outlined in Example 9, (-)-trans-4-(4'-fluorophenyl)-3-(3',4'methylenedioxyphenoxymethyl)piperidine hydrochloride (2.00g) was converted to the title compound E14 (0.73g, 28%) m.p. 210-12° (ethanol-ether)

nmr ($D_6$ DMSO, δ)

1.80-2.02 (m, 1H)
2.27-2.55 (m, 1H)
2.70-3.00 (m, 2H)
3.00-3.85 (m, 6H)
4.34-4.60,4.75-4.90 (2 x m, 2H)
5.95 (s, 2H)
6.21 (dd, 1H)
6.49 (d, 1H)
6.76 (d, 1H)
7.05-7.46 (m, 6H)
7.46-7.70 (m, 1H)
7.80-8.03 (m, 1H)
11.92 (br.s, 1H)

Example 15

(-) trans-1-Benzyl-3-(3',4'-methylenedioxyphenoxy-methyl)-4-phenylpiperidine hydrochloride (E15)

(-) trans

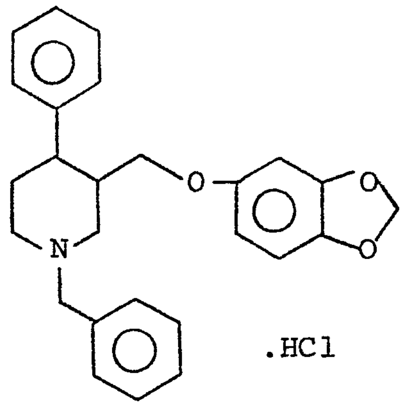

.HCl

(E15)

Following the general method outlined in Example 9, (-) trans-3-(3',4'-methylenedioxyphenoxymethyl)-4-phenylpiperidine (resolved D32) (1.29g) was converted to the title compound E15 (0.60g, 37%) m.p. 219-23$^{\circ}$ (ethanol-ether).

nmr ($D_6DMSO$, $\delta$)

1.78-2.00 (m, 1H)
2.20-2.48 (m, 1H)
2.67-2.92 (m, 2H)
2.92-3.20 (m, 2H)
3.22-3.77 (m, 4H)
4.25-4.56,4.60-4.80 (2 x m, 2H)
5.92 (s, 2H)
6.15 (dd, 1H)
6.45 (d, 1H)
6.72 (d, 1H)
7.10-7.40 (m, 5H)
7.40-7.55 (m, 3H)
7.60-7.85 (m, 2H)
11.65 (br.s, 1H)

Example 16

(-) trans-1-(4'-Fluorobenzyl)-3-(3',4'-methylenedioxy-phenoxymethyl)-4-phenylpiperidine hydrochloride (E16)

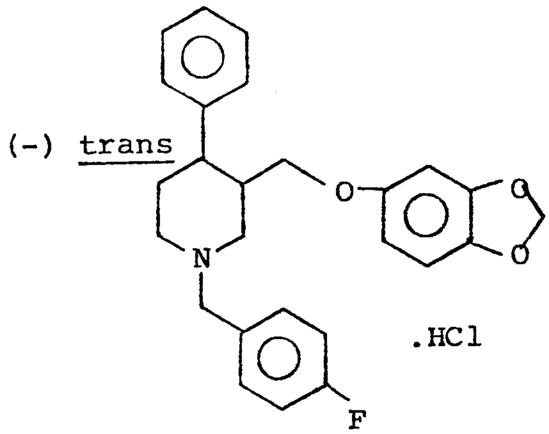

(E16)

Following the general method outlined in Example 9, (-) trans-3-(3',4'-methylenedioxyphenoxymethyl)-4-phenylpiperidine (resolved D32) (1.90g) was converted to the title compound E16 (0.60g, 24%) m.p. 223-27$^{o}$ (ethanol-ether).

nmr ($D_6$ DMSO, δ)

1.77-2.00 (m, 1H)
2.20-2.46 (m, 1H)
2.65-2.90 (m, 2H)
2.90-3.18 (m, 2H)
3.20-3.50 (m, 2H)
3.50-3.70 (m, 2H)
4.25-4.55,4.60-4.78 (2 x m, 2H)
5.92 (s, 2H)
6.17 (dd, 1H)
6.46 (d, 1H)
6.71 (d, 1H)
7.12-7.50 (m, 7H)
7.65-7.95 (m, 2H)
11.56 (br.s, 1H)

Example 17

(±)-trans-1-Benzyl-3-(3',4'-methylenedioxyphenoxymethyl)-4-(2'-methylphenyl)piperidine hydrochloride (E17)

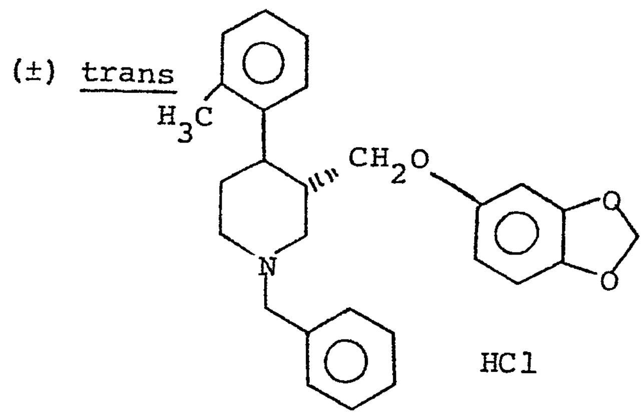

(E17)

Following the general method outlined in Example 9, except that benzyl chloride was substituted for 4-fluorobenzyl chloride, the amine D38 (0.49g) was converted the title compound E17 (0.348g, 57%) m.p. 221-27° (ethanol-ether) as white needles.

n.m.r. ($D_6$-DMSO. δ)

1.80-1.96 (m, 1H)
2.10-2.40 (m, 1H)
2.27 (s, 3H)
2.80-3.24 (m, 4H)
3.24-3.50 (m, 1H)
3.40 (s, 3H)
3.50-3.70 (m, 2H)
5.94 (s, 2H)
6.17 (dd, 1H)
6.45 (d, 1H)
6.84 (d, 1H)
7.00-7.35 (m, 4H)
7.40-7.58 (m, 3H)
7.62-7.85 (m, 2H)

Example 18

(±)-trans-1-Benzyl-4-(4'-fluorophenyl)-3-(4'-methylphenoxymethyl)piperidine maleate (E18)

(±) trans

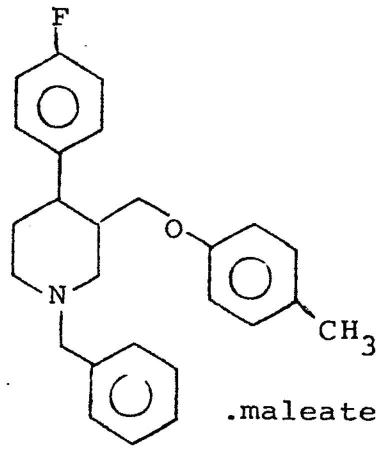

.maleate

(E18)

Following the general method outlined in Example 9, except that benzyl chloride was substituted for 4-fluorobenzyl chloride, (±)-trans-4-(4'-fluorophenyl)-3-(4'-methylphenoxymethyl)piperidine hydrochloride D39 (1.03g) was converted to the title compound E18 (0.194g, 13%), as a white solid, m.p. 146-8° (methanol).

nmr ($CDCl_3$, δ)

1.95-2.10 (m, 1H)
2.20-2.45 (m, 1H)
2.25 (s, 3H)
2.50-2.70 (m, 1H)
2.70-3.10 (m, 3H)
3.40-3.80 (m, 4H)
4.15-4.40 (m, 2H)
6.40 (s, 2H)
6.62 (d, 2H)
6.90-7.20 (m, 6H)
7.45 (s, 5H)

Example 19

(±)-trans-1-(4'-Fluorobenzyl)-4-(4'-fluorophenyl)-3-(4'-methylphenoxymethyl)piperidine maleate (E19)

(±) trans

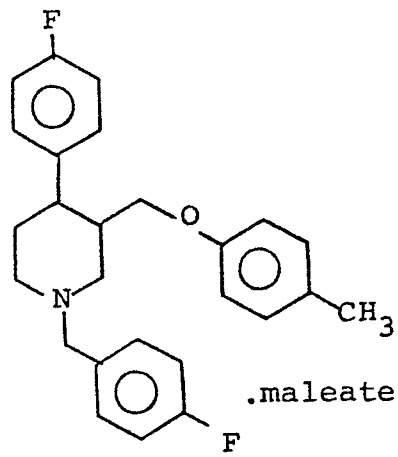

.maleate

(E19)

Following the general method outlined in Example 9, (±)-trans-4-(4'-fluorophenyl)-3-(4'-methylphenoxymethyl)piperidine hydrochloride D39 (1.03g) was converted to the title compound E19 (253mg, 16%), as a white solid, m.p. 126-7° (methanol).

nmr ($CDCl_3$, δ)

1.65 (brs, 1H)
1.75-2.30 (m, 5H)
2.25 (s, 3H)
2.40-2.58 (m, 1H)
2.90-3.05 (m, 1H)
3.15-3.30 (m, 1H)
3.40-3.75 (m, 4H)
6.55-6.65 (m, 2H)
6.90-7.65 (m, 11H)
7.90-8.00 (m, 1H)

Example 20

(±)-trans-1-(4'-Fluorobenzyl)-4-(4'-fluorophenyl)-3-phenoxymethylpiperidine maleate (E20)

(±) trans

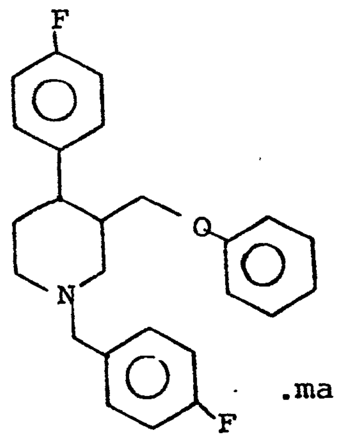

.maleate

(E20)

Following the general method outlined in Example 9, (±)-trans-4-(4'-fluorophenyl)-3-phenoxymethylpiperidine hydrochloride D40 (885mg) was converted to the title compound E20 (334mg, 24%), as a white solid, m.p. 142.5-144.5° (methanol).

nmr ($CDCl_3$, δ)

1.95-2.10 (m, 1H)
2.20-2.50 (m, 1H)
2.50-2.73 (m, 1H)
2.73-3.15 (m, 3H)
3.40-3.80 (m, 4H)
4.25 (s, 2H)
6.37 (s, 2H)
6.73 (d, 2H)
6.90-7.35 (m, 9H)
7.35-7.60 (m, 2H)

Example 21

(±)-trans-1-Benzyl-3-(4'-fluorophenoxymethyl)-4-(4'-fluorophenyl)piperidine maleate (E21)

(±) trans

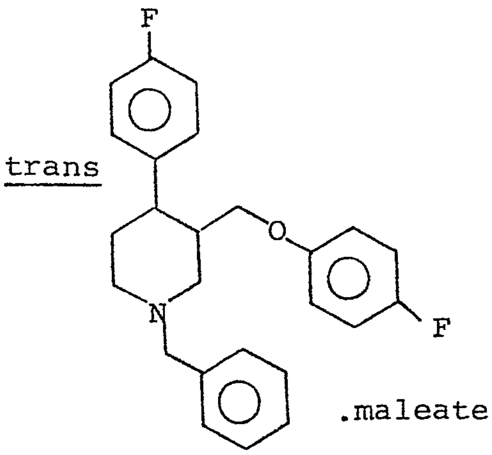

.maleate

(E21)

Following the general method outlined in Example 9, (±)-trans-3-(4'-fluorophenoxymethyl)-4-(4'-fluorophenyl)piperidine hydrochloride D41 (1.20g) was converted to the title compound E21 (350mg, 20%), as a white solid, m.p. 154-5° (methanol).

nmr ($CDCl_3$, δ)

1.95-2.10 (m, 1H)
2.20-2.45 (m, 1H)
2.50-2.70 (m, 1H)
2.70-3.10 (m, 3H)
3.40-3.55 (m, 1H)
3.55-3.80 (m, 3H)
4.15-4.40 (m, 2H)
6.38 (s, 2H)
6.55-6.75 (m, 2H)
6.80-7.05 (m, 4H)
7.05-7.20 (m, 2H)
7.40 (s, 5H)

Example 22

(±)-trans-1-(4'-Fluorobenzyl)-3-(4'-fluorophenoxymethyl)-4-(4'-fluorophenyl)piperidine maleate (E22)

(±) trans

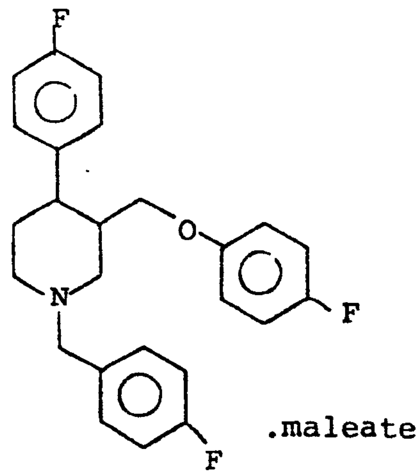

.maleate

(E22)

Following the general method outlined in example 9, (±)-trans-3-(4'-fluorophenoxymethyl)-4-(4'-fluorophenyl)piperidine hydrochloride D41 (1.20g) was converted to the title compound E22 (372mg, 20%), as a white solid, m.p. 172-4° (methanol).

nmr ($CDCl_3$, δ)

2.00-2.35 (m, 2H)
2.45-2.65 (m, 1H)
2.85-3.20 (m, 3H)
3.45-3.85 (m, 4H)
4.35 (q, 2H)
6.35 (s, 2H)
6.60-6.75 (m, 2H)
6.85-7.10 (m, 4H)
7.10-7.30 (m, 4H)
7.45-7.60 (m, 2H)

Examples 23 and 24

The following compounds are prepared analogously:

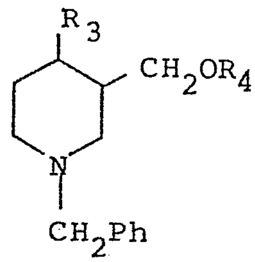

(E23 & 24)

| Example No. | R3 | R4 | Isomer |
|---|---|---|---|
| 23 | 3,4-$OCH_2O$-Ph | 4-F-Ph | (±)*trans* |
| 24 | 4-F-Ph | 3-$CF_3$-Ph | (-)*trans* |

## Pharmacological Data Section

## ANTIULCER TESTS

### 1. Cold restraint stress - induced gastric erosions

Male Wistar rats, 175-225g bodyweight are used. Forty eight hours prior to testing, animals are placed in mesh-bottomed cages with 10-12 animals per cage. Twenty-four hours prior to testing, animals are placed in the dark at a temperature of 17°C. Sixteen hours prior to testing, food is removed from the cages. Drugs are made up in 0.5% methylcellulose, and adminstered at a volume of 1.0ml/100g bodyweight. Eight rats per group are used, with usually 1 control group and 2 test groups. Animals are placed in Bollman type restraining cages and arranged in a vertical position. Thirty minutes after dosing the animals are placed in an ambient temperature of 4°C for 2.5 hours. At the end of this time the rats are sacrificed, the stomachs removed, cut open and pinned out on cork boards. Mucosal damage is assessed visually and scored on a subjective scale. Statistical differences between control and test groups are assessed using a Wilcoxon Ranking Test. The results are shown in Table 1.

Table 1:

| Compound | Oral Dose µmol/kg (mg/kg free base) | % Inhibition of gastric erosions |
|---|---|---|
| Example 1 | 25 (10.5) | 53** |
| Example 2 | 25 (11.2) | 58* |

 * $p<0.05$
** $p<0.01$

2. Inhibition of ethanol-induced gastric mucosal erosive damage in the rat

The object of the experiment was to test the ability of drugs to inhibit a state of gastric submucosal haemorrhage induced by absolute ethanol in the conscious rat. Inhibition of this mucosal damage by drugs may be of use in the development of anti-ulcer agents.

The procedure used was as follows. Male rats, preferably Wistars, were fasted overnight in wire mesh bottom cages, so as to prevent copraphagy. Water was allowed ad. libitum. Six or seven rats per cage were regarded as suitable, and the weight range of the rats used was between 175 and 300g. Water was removed one hour prior to treatment. The rats were then randomly allocated to their respective treatment groups, so that there were no less than five rats per group. One control group was always included.

Each rat was pre-dosed with 1ml/100g of control or test drug, dissolved or suspended in 0.5% methyl cellulose or 1% Tween 80 in water, via an oral dosing needle. Ninety minutes later each rat was dosed with 1ml/rat p.o. of absolute ethanol. Ten minutes after the absolute ethanol, the rats were killed by an intracardiac injection of Expiral*. The stomachs were excised, cut open along the greater curvature to expose the mucosal surface, washed in tap water to remove debris, then stretched on a cork board for examination.

A subjective macroscopic examination of the stomach was carried out with a damage score being allocated to each stomach. Two observers were usually used, one of whom scored the results in a 'blinded' manner. The results were then analysed by the Mann-Whitney U-test, with $p<0.05$ being regarded as statistically significant. The results as shown in Table 2.

*(sodium pentobarbitone solution 200mg/ml)

Table 2:

| Compound | Oral Dose µmol/kg | (mg/kg free base) | % Inhibition of gastric erosions |
|---|---|---|---|
| Example 1 | 25 | (10.47) | 66*** |
| Example 2 | 25 | (10.75) | 47.7** |
| Example 5 | 25 | (10.47) | 30** |
| Example 7 | 25 | (10.47) | 63.1** |
| Example 8 | 25 | (10.47) | 46.8* |
| Example 9 | 25 | (10.92) | 71*** |
| Example 11 | 25 | (10.42) | 26** |
| Example 12 | 25 | (10.92) | 84.9** |
| Example 13 | 25 | (10.92) | 46** |
| Example 14 | 25 | (10.92) | 39* |
| Example 15 | 25 | (10.02) | 67** |
| Example 16 | 25 | (10.47) | 83** |
| Example 17 | 25 | (10.37) | 31* |
| Example 18 | 25 | (9.72) | 21* |
| Example 19 | 25 | (10.17) | 69** |
| Example 20 | 25 | (9.82) | 70** |
| Example 21 | 25 | (9.82) | 27* |
| Example 22 | 25 | (10.27) | 53* |

* $p<0.05$

** $p<0.01$

*** $p<0.001$

## GASTRO-INTESTINAL MOTILITY TESTS

### Intraluminal pressure in the Heidenhain pouch of the dog.

Pressure changes were recorded via a saline filled catheter inserted, with airtight closure, into the fistula of a chronic Heidenhain pouch of the previously fasted and lightly restrained conscious dog. The catheter was connected to a physiological pressure transducer and pressure changes recorded on a hot wire pen recorder. Compounds were administered when the motility was in a phase of relatively low activity and the dose range determined which induced an increase in the amplitude of rhythmical contractions for a period of at least 4-5 minutes.

The Compound of Example 1 was active at a dose of 0.5mg/kg i.v.

Claims

1. A compound of formula (I) or a pharmaceutically acceptable salt thereof:

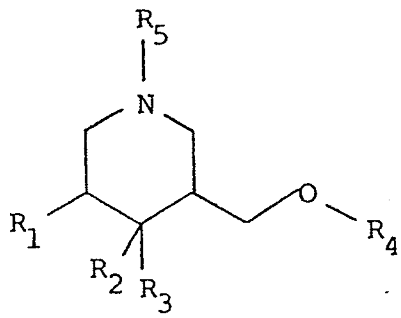

(I)

wherein

$R_1$ and $R_2$ are both hydrogen or together are a bond;

$R_3$ and $R_4$ are independently optionally substituted phenyl or naphthyl groups;

$R_5$ is a group $(CH_2)_nR_6$ wherein n is 1 or 2 and $R_6$ is an optionally substituted phenyl or naphthyl group.

2. A compound according to claim 1 wherein $R_1$ and $R_2$ are both hydrogen.

3. A compound according to claim 1 or 2 wherein $R_3$, $R_4$ and $R_6$ are phenyl or naphthyl optionally substituted by one, two or three groups independently selected from halogen, $CF_3$, $C_{1-6}$ alkoxy, $C_{1-6}$ alkylthio, $C_{1-6}$ alkyl, nitro, cyano, carboxyl, hydroxy, $C_{1-6}$ alkoxycarbonyl, $C_{1-10}$ carboxylic acyl, and amino optionally substituted by one or two $C_{1-6}$ alkyl groups, disubstituted by $C_{3-6}$ polymethylene optionally containing oxygen, sulphur or

$NR_7$ wherein $R_7$ is hydrogen or $C_{1-6}$ alkyl, or monosubstituted by $C_{1-4}$ alkanoyl; or $R_3$, $R_4$ and/or $R_6$ is/are disubstituted on adjacent carbon atoms by methylenedioxy, ethylenedioxy, $C_{3-5}$ polymethylene or $-CH=CH-(CH_2)_2-$.

4. A compound according to claim 3 wherein $R_3$ is phenyl monosubstituted by halogen or methoxy.

5. A compound according to claim 3 wherein $R_4$ is phenyl 3,4-disubstituted by methylenedioxy or $R_4$ is 2-methoxyphenyl.

6. A compound according to claim 3 wherein $R_5$ is $CH_2R_6$ wherein $R_6$ is unsubstituted phenyl or phenyl monosubstituted by nitro or halo.

7. A compound according to claim 6 wherein $R_6$ is 4-fluorophenyl.

8. (-)-trans-1-Benzyl-4-(4'-fluorophenyl)-3-(3',4'-methylenedioxyphenoxymethyl)piperidine,

(±)trans 1-benzyl-4-(2'-methoxyphenyl)-3-(3',4'-methylenedioxyphenoxymethyl)piperidine,

(-)trans-1-benzyl-4-(4'-fluorophenyl)-3-(2'-methoxy-phenoxymethyl)piperidine,

(-)trans-4-(4'-fluorophenyl)-3-(3',4'-methylenedioxy-phenoxymethyl)-1-(4'-nitrobenzyl) piperidine,

(+)trans-1-benzyl-4-(4'-fluorophenyl)-3-(3',4'-methylenedioxyphenoxymethyl)piperidine,

(-)-trans-4-(4'-fluorophenyl)-3-(3',4'-methylenedioxy-phenoxymethyl-1-((2'-ethyl)phenyl) piperidine,

(+)cis-1-benzyl-4-(4'-fluorophenyl)3-(3',4'-methylene-dioxyphenoxymethyl)piperidine,

(-)cis-1-benzyl-4-(4'-fluorophenyl)-3-(3',4'-methylene-dioxyphenoxymethyl)piperidine,

(-) trans-1-(4'-fluorobenzyl)-4-(4'-fluorophenyl)-3-(3',4'-methylenedioxyphenoxymethyl) piperidine,

(-)-trans-1-(4'-chlorobenzyl)-4-(4'-fluorophenyl)-3-(3',4'-methylenedioxyphenoxymethyl) piperidine,

(±)-1-benzyl-4-(4'-fluorophenyl)-3-(3,4-methylenedioxy-phenoxymethyl)-1,2,3,6-tetrahydropyridine,

(+) cis-1-(4'-fluorobenzyl)-4-(4'-fluorophenyl) -3-(3', 4'-methylenedioxyphenoxymethyl)piperidine,

(-) cis-1-(4'-fluorobenzyl)-4-(4'-fluorophenyl) -3-(3', 4'-methylenedioxyphenoxymethyl)piperidine,

(-) trans-1-(2'-fluorobenzyl)-4-(4'-fluorophenyl)-3-(3',4-methylenedioxyphenoxymethyl) piperidine,

(-) <u>trans</u>-1-benzyl-3-(3',4'-methylenedioxyphenoxy- methyl)-4-phenylpiperidine,

(-) <u>trans</u>-1-(4'-fluorobenzyl)-3-(3',4'-methylenedioxy-phenoxymethyl)-4-phenylpiperidine,

(±)-<u>trans</u>-1-benzyl-3-(3',4'-methylenedioxy-phenoxy methyl)-4-(2'-methylphenyl)piperidine,

(±)-<u>trans</u>-1-benzyl-4-(4'-fluorophenyl)-3-(4'-methyl-phenoxymethyl)piperidine,

(±)-<u>trans</u>-1-(4'-fluorobenzyl)-4-(4'-fluorophenyl)-3-(4'-methylphenoxymethyl)piperidine,

(±)-<u>trans</u>-1-(4'-fluorobenzyl)-4-(4'-fluorophenyl)-3-phenoxymethylpiperidine,

(±)-<u>trans</u>-1-benzyl-3-(4'-fluorophenoxymethyl)-4-(4'-fluorophenyl)piperidine,

(±)-<u>trans</u>-1-(4'-fluorobenzyl)-3-(4'-fluorophenoxy-methyl)-4-(4'-fluorophenyl)piperidine,

or a pharmaceutically acceptable salt of any of the foregoing.

9. A process for the preparation of a compound according to any one of claims 1 to 8 which process comprises reacting a compound of formula (II)

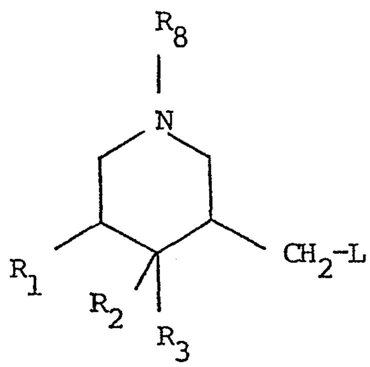

(II).

wherein:

L is a leaving group or $OR_4$;

$R_8$ is hydrogen when L is $OR_4$ or $(CH_2)_nR_6$ when L is a leaving group; and $R_1$, $R_2$ and $R_3$ are as defined in claim 1; with

i) $R_6(CH_2)_nQ$ wherein Q is a leaving group when $R_8$ is hydrogen); or

ii) $R_4$ OH or an alkali metal salt thereof (when L is a leaving group);

and thereafter optionally converting substituents in $R_3$, $R_4$ and/or $R_6$ to other substituents in $R_3$, $R_4$ and/or $R_6$ and/or forming a pharmaceutically acceptable salt.

10. A compound of formula (III) and pharmaceutically acceptable salts thereof:

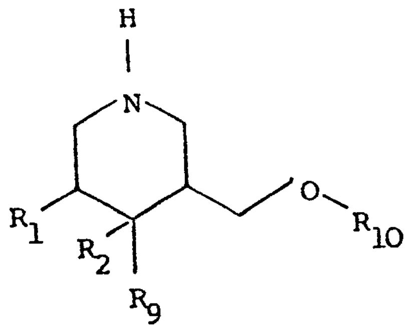

(III)

wherein:

$R_9$ is 3,4-methylenedioxyphenyl and $R_{10}$ is 4-fluorophenyl; or

$R_9$ is 4-fluorophenyl and $R_{10}$ is phenyl, 4-fluorophenyl, 2-methoxyphenyl, 4-methylphenyl, 3-trifluoromethylphenyl; or

$R_9$ is phenyl, 2-methoxyphenyl or 2-methylphenyl and $R_{10}$ is 3,4-methylenedioxyphenyl; and

$R_1$ and $R_2$ are as defined in claim 1.

11. A pharmaceutical composition comprising a compound according to any one of claims 1 to 8 or a pharmaceutically acceptable salt thereof, and a pharmaceutically acceptable carrier.

12. A compound according to any one of claims 1 to 8 or a pharmaceutically acceptable salt thereof for use as an active therapeutic substance.

13. A compound according to any one of claims 1 to 8 or a pharmaceutically acceptable salt thereof for use in treatment of disorders relating to damaged gastro-intestinal tissue and to impaired gastro-intestinal motility.